(19) Europäisches Patentamt
European Patent Office
Office européen des brevets

(11) **EP 3 230 448 B1**

## (12) EUROPEAN PATENT SPECIFICATION

(45) Date of publication and mention
of the grant of the patent:
**10.04.2019 Bulletin 2019/15**

(21) Application number: **15823730.5**

(22) Date of filing: **09.12.2015**

(51) Int Cl.:
*C12N 15/10* [(2006.01)]     *C12M 1/12* [(2006.01)]

(86) International application number:
**PCT/GB2015/053774**

(87) International publication number:
**WO 2016/092304 (16.06.2016 Gazette 2016/24)**

## (54) METHOD FOR SCREENING FOR BIOACTIVE NATURAL PRODUCTS

VERFAHREN ZUM SCREENING FÜR BIOAKTIVE NATÜRLICHE PRODUKTE

PROCÉDÉ DE CRIBLAGE DE PRODUITS NATURELS BIOACTIFS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.12.2014 GB 201421850**

(43) Date of publication of application:
**18.10.2017 Bulletin 2017/42**

(73) Proprietor: **Nanna Therapeutics Limited**
**Cambridge**
**Cambridgeshire CB1 3LQ (GB)**

(72) Inventors:
• **WILLIAMS, David Hugh**
**Cambridge**
**Cambridgeshire CB1 3LQ (GB)**
• **WAIN, John Richard**
**Cambridge**
**Cambridgeshire CB1 3LQ (GB)**
• **WOODS, Stuart Robert**
**Cambridge**
**Cambridgeshire CB1 3LQ (GB)**

(74) Representative: **Hutchins, Michael Richard**
**Schlich**
**9 St Catherine's Road**
**Littlehampton, West Sussex BN17 5HS (GB)**

(56) References cited:
**WO-A1-2014/072697    GB-A- 2 505 819**

• **LI CHEN ET AL:** "Transposon activation mutagenesis as a screening tool for identifying resistance to cancer therapeutics", BMC CANCER, BIOMED CENTRAL, LONDON, GB, vol. 13, no. 1, 27 February 2013 (2013-02-27), page 93, XP021141268, ISSN: 1471-2407, DOI: 10.1186/1471-2407-13-93
• **GEMMA C LANGRIDGE ET AL:** "Simultaneous assay of every Salmonella Typhi gene using one million transposon mutants", GENOME RESEARCH, COLD SPRING HARBOR LABORATORY PRESS , vol. 19, no. 12 1 December 2009 (2009-12-01), pages 2308-2316, XP002682200, ISSN: 1088-9051, DOI: 10.1101/GR.097097.109 Retrieved from the Internet: URL:http://genome.cshlp.org/content/19/12/2308 [retrieved on 2009-10-13]
• **AHARONI A ET AL:** "High-Throughput Screening of Enzyme Libraries: Thiolactonases Evolved by Fluorescence-Activated Sorting of Single Cells in Emulsion Compartments", CHEMISTRY AND BIOLOGY, CURRENT BIOLOGY, LONDON, GB, vol. 12, no. 12, 1 December 2005 (2005-12-01), pages 1281-1289, XP027681876, ISSN: 1074-5521 [retrieved on 2005-12-01]
• **BERNATH K ET AL:** "In vitro compartmentalization by double emulsions: sorting and gene enrichment by fluorescence activated cell sorting", ANALYTICAL BIOCHEMISTRY, ACADEMIC PRESS INC, NEW YORK, vol. 325, no. 1, 1 February 2004 (2004-02-01), pages 151-157, XP004483545, ISSN: 0003-2697, DOI: 10.1016/J.AB.2003.10.005

- BARET JEAN-CHRISTOPHE ET AL: "Fluorescence-activated droplet sorting (FADS): efficient microfluidic cell sorting based on enzymatic activity", LAB ON A CHIP, ROYAL SOCIETY OF CHEMISTRY - CAMBRIDGE, GB , vol. 9, no. 13 7 July 2009 (2009-07-07), pages 1850-1858, XP009122819, ISSN: 1473-0197, DOI: 10.1039/B902504A Retrieved from the Internet: URL:http://www.rsc.org/Publishing/Journals /lc/Index.asp

**Description**

The present invention relates to methods for screening mutant prokaryotic cells to identify producers of cytotoxic agents (such as antibiotics and anticancer agents) active against a target cell (such as pathogenic bacteria and tumour cells), and to methods of identifying a cytotoxic agent comprising such screening methods. The invention also relates to processes for producing a cytotoxic agent comprising the methods of the invention.

Bacteria are a major source of bioactive natural products, including antibiotics, anticancer agents, crop protection agents and immunosuppressants. For example, actinobacteria, especially *Streptomyces* spp., are producers of many bioactive secondary metabolites that are useful in medicine (e.g. as antibacterials, antifungals, antivirals, antithrombotics, immunomodulatory agents, anticancer agents and enzyme inhibitors) and in agriculture (e.g. as insecticides, herbicides, fungicides and growth promoting substances for plants and animals). Actinobacteria-derived antibiotics that are important in medicine include aminoglycosides, anthracyclines, chloramphenicol, macrolide and tetracyclines, while natural bacterial products such as bleomycin, doxorubicin, rapamycin and mithramycin are the basis of important anticancer therapeutics.

However, there is an urgent need for new cytotoxic agents, especially antibiotics, to counter the emergence of new pathogens and resistance to existing antimicrobial drugs, whilst the range of anticancer agents must be expanded.

Traditional screening for producers of cytotoxic agents include testing pure strains of a candidate producer for activity against target cells in solid or liquid media. In the former case, individual producing bacteria with genetic mutants can be plated onto lawns of target cells so that those producing the desired cytotoxic agents can be identified by the appearance of zones of inhibition/clearing surrounding the emergent mutant colonies. However, this technique is laborious, cannot typically be applied in the case of mammalian target cells, and is not suited to high throughput screens.

WO 2014/072697 describes a process for producing a mutant bacterium which exhibits improved survival and/or growth under a selected growth conditions comprising the steps of: (a) generating a pool of mutant bacteria by transposon mutagenesis with an activating transposon (TnA), wherein the TnA comprises a promoter capable of increasing transcription of a gene at or near its insertion site; (b) growing bacteria from the mutant pool under the selected growth condition and under one or more reference conditions to produce two or more test cultures; and (c) comparing the distribution of TnA insertions between test cultures to identify a first class of genes which are disadvantageous for growth and/or survival under the selected growth condition and a second class of genes which are advantageous for growth and/or survival under the selected growth condition.

GB 2 505 819 describes a method for identifying genes mediating antibiotic sensitivity or resistance, wherein the method utilises transposons with three different promoters of varying strengths to generate mutant bacteria. The method comprises the steps of generating a pool of mutant bacteria by transposon mutagenesis of Gram-negative bacterium with a plurality of activating transposons (TnA), each transposon comprising an outward-facing promoter (TnP); growing bacteria from the mutant pool in the presence of different amounts of said antibiotic; and comparing the distribution of TnA insertions between test cultures to identify a gene which mediates antibiotic sensitivity or resistance in the bacterium.

Aharoni et al: "High-Throughput Screening of Enzyme Libraries: Thiolactonases Evolved by Fluorescence-Activated Sorting of Single Cells in Emulsion Compartments", Chemistry and Biology, vol. 12, no. 12, pages 1281-1289, describes single bacterial cells, each expressing a different library variant compartmentalized in aqueous droplets of water-in-oil (w/o) emulsions, thus maintaining a linkage between a plasmid-borne gene, the encoded enzyme variant, and the fluorescent product this enzyme may generate. Conversion into a double, water-in-oil-in-water (w/o/w) emulsion enables the sorting of these compartments by FACS, as well as the isolation of living bacteria cells and their enzyme-encoding genes.

Bernath et al: "In vitro compartmentalization by double emulsions: sorting and gene enrichment by fluorescence activated cell sorting", Analytical Biochemistry, vol. 325, no. 1, pages 151-157, describes water-in-oil (w/o) emulsions that can be used to compartmentalize and select large gene libraries for a predetermined function. The aqueous droplets of the w/o emulsion function as cell-like compartments in each of which a single gene is transcribed and translated to give multiple copies of the protein (e.g., an enzyme) it encodes. Re-emulsification of w/o emulsions gives water-in-oil-in-water (w/o/w) emulsions with an external (continuous) water phase through which droplets containing fluorescent markers can be isolated by fluorescence-activated cell sorting (FACS).

Baret et al: "Fluorescence-activated droplet sorting (FADS): efficient microfluidic cell sorting based on enzymatic activity", Lab on a Chip, vol. 9, no. 13, pages 1850-1858, describes a microfluidic fluorescence-activated droplet sorter (FADS) combining microtitre-plate screening and traditional fluorescence-activated cell sorting (FACS).

Screening mutants in liquid media is complicated by the facts that mutants of interest producing cytotoxic compounds may exhibit widely different growth rates, greatly reducing the diversity of the recovered producer mutants. Moreover, target mutants producing cytotoxic compounds may also be overgrown by "cheaters", these being mutants which are resistant to the cytotoxic compounds produced by the target mutant producer cells but which do not themselves produce the cytotoxic agent (so enjoying a metabolic advantage reflected in a higher growth rate).

A further problem associated with screening for producer mutants in liquid culture arises from the fact that the cytotoxic compounds of interest may be produced at relatively low concentrations, and so effectively diluted out by the bulk liquid culture medium. Thus, valuable signals arising from mutant producer cells may go undetected (or be obscured by the effects of mutant producer cells secreting more potent cytotoxic agents).

A major challenge to the development of new bioactive natural products is therefore the need to screen large numbers of producer bacteria to identify those elaborating products having the desired activity, coupled with the need to secure a source of rich biological diversity at the level of the candidate producer organisms to be screened. There is also a need for methods which can be used in high-throughput, massively parallel screens so that very large numbers of different producer cell mutants can be screened against a wide range of different target cells.

According to an aspect of the present invention, there is provided a method for screening mutant prokaryotic cells to identify producers of a cytotoxic agent active against a target cell, the method comprising the steps of:

(a) providing cells of a producer prokaryotic species;
(b) generating a pool of mutant producer cells by transposon mutagenesis of the cells of step (a) with an activating transposon ($Tn_A$), wherein the $Tn_A$ comprises an outward-facing promoter ($Tn_AP$) capable of increasing transcription of a gene at or near its insertion site in the DNA of said producer cells;
(c) co-encapsulating individual members of the pool of step (b) with one or more target cells in microdroplets, the microdroplets comprising a volume of aqueous growth media suspended in an immiscible carrier liquid, thereby generating a library of microdroplets each comprising a single mutant producer cell and one or more target cell(s);
(d) incubating the microdroplet library of step (c) under conditions suitable for co-culture of the single mutant producer cell and target cell(s) to produce a library of microcultures, whereby mutant producer cells producing a cytotoxic agent active against the target cell(s) outgrow target cells in each microculture; and
(e) screening the library of microcultures of step (d) for microcultures in which target cells have been outgrown or overgrown to extinction by mutant producer cells.

The encapsulation step is preferably conducted such that each microdroplet contains a single member of the mutant pool, together with two or more (for example, about 10) target cells. Depending on the encapsulation process employed, the microdroplet library may be heterogeneous with respect to cellular content, and some microdroplets may be empty. However, all that is required is that encapsulation result in the recovery of at least some microdroplets containing a single mutant producer cell from the mutant pool along with one or more target cells.

The method of the invention permits enrichment for microcultures in which target cells have been killed and/or outgrown by mutant producer cells, which mutant cells may be subsequently isolated and analysed to identify the basis for their cytotoxic activity. Since the assay is performed in relatively small volumes, the effect of each cytotoxic compound produced by a mutant producer cell may be detected without the diluting effect of a large volume of media and/or the confounding effect of other (possibly more potent) cytotoxic agents produced by other mutant producer cells. Thus, the method is much more sensitive to signals generated by mutant producer cells.

The method also avoids the "swamping" effect of "cheaters", these being mutant producer cells which are resistant to the cytotoxic compounds produced by the target mutant producer cells but which do not themselves produce the cytotoxic agent (so enjoying a metabolic advantage reflected in a higher growth rate). Such "cheaters" would overgrow and effectively extinguish the signal generated by mutant producers of cytotoxic agents if not effectively partitioned from individual producer cell mutants by the encapsulation step of the invention.

The method may further comprise sequencing the DNA of mutant producer cells in microdroplets in which target cells have been outgrown or overgrown to extinction by mutant producer cells during the incubation step. Such microdroplets can be isolated by various sorting techniques (see infra), and the cells can be released from the microdroplets by any convenient method (for example by the addition of surfactants, detergents, by sonication, by osmotic shock or by mechanical or physicochemical means).

DNA adjacent or near the insertion site of the $Tn_A$ is preferably sequenced, for example by methods comprising the selective amplification of transposon-cellular DNA junctions.

In preferred embodiments, the sequencing comprises high-throughput massively parallel sequencing. Any such type of sequencing may be employed, for example selected from: (a) sequencing-by-synthesis (SBS) biochemistry; and/or (b) nanopore sequencing; and/or (c) tunnelling current sequencing; and/or (d) pyrosequencing; and/or (e) sequencing-by-ligation (SOLiD sequencing); and/or (f) ion semiconductor; and/or (g) mass spectrometry sequencing.

Preferably, about 25, 50, 75, 100 or greater than 100 base pairs of DNA adjacent or near the $Tn_A$ insertion site are sequenced. The sequenced DNA may be 5' and/or 3' to the $Tn_A$ insertion site.

The methods of the invention may further comprise the step of sequencing mRNA transcripts produced by $Tn_AP$ in mutant producer cells in microdroplets in which target cells have been outgrown or overgrown to extinction by mutant producer cells to produce an mRNA transcript profile. In such embodiments, the mRNA transcript profile comprises a determination of:

(a) the sequences of said mRNA transcripts produced by $Tn_AP$; and/or

(b) the start and finish of mRNA transcripts produced by TnAP; and/or

(c) the lengths of said mRNA transcripts produced by $Tn_AP$; and/or

(d) the relative abundance of said mRNA transcripts produced by $Tn_AP$; and/or

(e) the site of transcription on the cellular DNA; and/or

(f) whether the mRNA transcripts produced by $Tn_AP$ is sense or antisense with respect to the cellular DNA; and/or

(g) whether the mRNA transcripts produced by $Tn_AP$ correspond to ORFs with respect to the cellular DNA; and/or

(h) whether the mRNA transcripts produced by $Tn_AP$ encode prokaryotic proteins and/or protein domains.

The size of the microdroplets will be selected by reference to the nature of the cells (both producer and target) to be encapsulated, and the number of doublings to be achieved during the incubation step. Typically, the microdroplets are sized to provide a volume of growth medium sufficient to support 1000 cells. Thus, the microdroplets may be substantially spherical with a diameter of: (a) 10 $\mu$m to 500 $\mu$m; (b) 10 $\mu$m to 200 $\mu$m; (c) 10 $\mu$m to 150 $\mu$m; (d) 10 $\mu$m to 100 $\mu$m; (e) 10 $\mu$m to 50 $\mu$m; or (f) about 100 $\mu$m.

While the microdroplets may comprise a volume of aqueous growth media in the gel state, in preferred embodiments they comprise a volume of aqueous growth media in the liquid state. In such embodiments, the microdroplets may comprise an inner core of aqueous growth media enveloped in an outer oil shell, the carrier liquid being a continuous aqueous phase. Here, the inner aqueous core has a diameter of: (a) 10 $\mu$m to 500 $\mu$m; (b) 10 $\mu$m to 200 $\mu$m; (c) 10 $\mu$m to 150 $\mu$m; (d) 10 $\mu$m to 100 $\mu$m; (e) 10 $\mu$m to 50 $\mu$m; or (f) about 100 $\mu$m, while the outer oil shell may have a thickness of: (a) 10 $\mu$m to 200 $\mu$m; (b) 10 $\mu$m to 200 $\mu$m; (c) 10 $\mu$m to 150 $\mu$m; (d) 10 $\mu$m to 100 $\mu$m; (e) 10 $\mu$m to 50 $\mu$m; or (f) about 100 $\mu$m.

In single W/O type emulsions, the carrier liquid may be any water-immiscible liquid, for example an oil, optionally selected from: (a) a hydrocarbon oil; (b) a fluorocarbon oil; (c) an ester oil; (d) an oil having low solubility for biological components of the aqueous phase; (e) an oil which inhibits molecular diffusion between microdroplets; (f) an oil which is hydrophobic and lipophobic; (g) an oil having good solubility for gases; and/or (h) combinations of any two or more of the foregoing.

Thus, the microdroplets may be comprised in a W/O emulsion wherein the microdroplets constitute an aqueous, dispersed, phase and the carrier liquid constitutes a continuous oil phase.

In other embodiments, the microdroplets are comprised in a W/O/W double emulsion and the carrier liquid may an aqueous liquid. In such embodiments, the aqueous liquid may be phosphate buffered saline (PBS).

The microdroplets may therefore be comprised in a W/O/W double emulsion wherein the microdroplets comprise: (a) an inner core of aqueous growth media enveloped in an outer oil shell as the dispersed phase, and (b) the carrier liquid as the continuous aqueous phase.

In embodiments where the microdroplets are comprised in an emulsion, the carrier liquid may constitute the continuous phase and the microdroplets the dispersed phase, and in such embodiments the emulsion may further comprise a surfactant and optionally a co-surfactant.

The surfactant and/or co-surfactant may be located at the interface of the dispersed and continuous phases, and when the microdroplets are comprised in a W/O/W double emulsion the surfactant and/or co-surfactant may be located at the interface of aqueous core and oil shell and at the interface of the oil shell and outer continuous phase The microdroplets may be monodispersed (as defined herein).

The co-encapsulation step may comprise mixing: (i) the pool of mutant producer cells; (ii) a population of the target cells; (iii) an aqueous growth medium; (iv) a water-immiscible liquid, for example an oil as defined herein; and (v) a surfactant, for example as defined herein, under conditions whereby a W/O type single emulsion comprising microdroplets of the aqueous growth medium dispersed in the water-immiscible liquid is formed.

In some embodiments, the W/O type single emulsion as described above is used for the incubation step. This may be preferred in circumstances where the continuous oil phase provides improved compartmentalization of the microcultures (for example, by preventing or limiting inter-culture interactions mediated by water soluble bioactive produces released during culture). In such embodiments, subsequent manipulation, screening (and in particular sorting) may be facilitated by a further, post-incubation, emulsification step wherein an aqueous carrier liquid, for example as defined herein, is mixed with the single emulsion used for the incubation step under conditions whereby a W/O/W double emulsion comprising microdroplets of the aqueous growth medium enveloped in the water-immiscible liquid and dispersed in the aqueous carrier liquid is formed.

The co-encapsulation step (c) may comprise mixing: (a) the pool of mutant producer cells; (b) a population of the target cells; (c) an aqueous growth medium; (d) a water-immiscible liquid, for example an oil as defined herein; (e) a surfactant, for example as defined herein, and (f) an aqueous carrier liquid, for example as defined herein, under conditions whereby a W/O/W double emulsion comprising microdroplets of the aqueous growth medium enveloped in the water-immiscible liquid and dispersed in the aqueous carrier liquid is formed.

Any means may be employed for the mixing step: for example, this step may comprise: (a) vortexing and/or (b) sonication; (c) homogenization; (d) pico-injection and/or (e) flow focusing.

As explained above, depending on the encapsulation process employed, the microdroplet library may be heterogeneous with respect to cellular content, and some microdroplets may be empty. In such circumstances the co-encapsulation step (c) may further comprise eliminating empty microdroplets which do not contain mutant producer and/or target cell(s). This step is conveniently achieved by Fluorescence-Activated Droplet Sorting (FADS), and in such embodiments the producer and/or target cells are fluorescently labelled.

The incubation step (d) is carried out for a period and under conditions selected by reference to the nature of the producer and target cells selected. Thus, this step may comprise maintaining the microdroplet library at a temperature of 15°C - 95°C for at least 1 hour. In some embodiments, the microdroplet library is maintained at a temperature of: (a) 15°C - 42°C; (b) 20°C - 40°C; (c) 20°C - 37°C; (d) 20°C - 30°C; or (e) about 25°C; (f) 40°C - 60°C; (g) 60°C - 80°C; or (h) 80°C - 98°C.

The incubation step (d) may comprise maintaining the microdroplet library at said temperature for about 2, 4, 6, 12, 24 or 48 hours, or for up to 7 days, for example for 1, 2, 3, 4, 5, 6 or 7 days. In other embodiments, the incubation step (d) comprises maintaining the microdroplet library at said temperature for up to 2 weeks, for example for 1 week or 2 weeks.

The screening step (e) may comprise eliminating microdroplets which contain target cells.

This is conveniently achieved by FADS, in which case the target cells may be fluorescently labelled. In this way, droplets containing only mutant producer cells which have outgrown or overgrown to extinction the target cells (or producer cells in co-culture with resistant producer cell mutants) can be isolated by sorting, thereby greatly enriching for mutant producer cells which elaborate cytotoxic agents against the target cells.

The method may further comprise a potency analysis step carried out during incubation step (d), whereby the relative growth rate in co-culture of mutant producer cells and target cells is determined. This may comprise sampling for microdroplets which contain only mutant producer cells by FADS during the incubation step, for example at different time points. Thus, two or more successive rounds of FADS selection may be carried out during incubation in order to recover different classes of producer mutants based on the potency of the cytotoxic agent produced. In such embodiments, target cells may be fluorescently labelled and microdroplets which contain target cells are subjected to continued incubation. Thus, the screening step (e) preferably comprises FADS sorting for microdroplets in which target cells have been outgrown or overgrown to extinction by mutant producer cells.

The producer prokaryotic species may be selected from archaea, for example selected from the phyla: (a) Crenarchaeota; (b) Euryarchaeota; (c) Korarchaeota; (d) Nanoarchaeota and (e) Thaumarchaeota, for example *Haloferax volcanii* or *Sulfolobus* spp. Alternatively, the producer prokaryotic species may be selected from bacteria, for example selected from: (a) actinomycetes; (b) *Pseudomonas* spp., and (c) *Bacillus* spp.

In preferred embodiments, the producer bacterial species is selected from *Streptomyces* spp., for example selected from: (a) *Streptomyces coelicolor,* (b) *Streptomyces lividans;* (c) *Streptomyces venezuealae*; (d) *Streptomyces griseus*; (e) *Streptomyces avermetilis;* and (f) *Streptomyces bingchenggensis*;

The target cell may be a bacterial or eukaryotic cell. For example, the target cell may be: (a) fungal; (b) mammalian; (c) a higher plant cell; (d) protozoal; (e) a helminth cell; (f) algal; or (h) an invertebrate cell.

In some embodiments, the target cell is a cancer cell, for example a human cancer cell.

In other embodiments, the target cell is a pathogenic bacterium.

In a second aspect, the invention provides a method of identifying a cytotoxic agent comprising screening mutant bacteria to identify producers of a cytotoxic agent active against a target cell according to a method as defined above.

In a third aspect, the invention provides a process for producing a cytotoxic agent comprising the method of the second aspect of the invention as defined above. Here, the process may further comprise synthesising or isolating said cytotoxic agent from the mutant bacteria, and may optionally further comprise mixing the synthesised or isolated cytotoxic agent with a pharmaceutically acceptable excipient to produce a pharmaceutical composition.

Other aspects and preferred embodiments of the invention are defined and described in the other claims set out below.

Where used herein and unless specifically indicated otherwise, the following terms are intended to have the following meanings in addition to any broader (or narrower) meanings the terms might enjoy in the art:

Unless otherwise required by context, the use herein of the singular is to be read to include the plural and *vice versa.* The term "a" or "an" used in relation to an entity is to be read to refer to one or more of that entity. As such, the terms "a" (or "an"), "one or more," and "at least one" are used interchangeably herein.

As used herein, the term "comprise," or variations thereof such as "comprises" or "comprising," are to be read to indicate the inclusion of any recited integer (e.g. a feature, element, characteristic, property, method/process step or limitation) or group of integers (e.g. features, element, characteristics, properties, method/process steps or limitations) but not the exclusion of any other integer or group of integers. Thus, as used herein the term "comprising" is inclusive or open-ended and does not exclude additional, unrecited integers or method/process steps.

The term *gene* is a term describing a hereditary unit consisting of a sequence of DNA that occupies a specific location on a chromosome or plasmid and determines a particular characteristic in an organism. A gene may determine a characteristic of an organism by specifying a polypeptide chain that forms a protein or part of a protein (structural gene); or encode an RNA molecule; or regulate the operation of other genes or repress such operation; or affect phenotype by some other as yet undefined mechanism.

The terms *genomic DNA* is a term of art used herein to define chromosomal DNA as distinct from extrachromosomally-maintained plasmid DNA.

The term *genome* is a term of art used herein to define the entire genetic complement of an organism, and so includes chromosomal, plasmid, prophage and any other DNA.

The term *Gram-positive bacterium* is a term of art defining a particular class of bacteria that are grouped together on the basis of certain cell wall staining characteristics.

The term *low G+C Gram-positive bacterium* is a term of art defining a particular subclass class of evolutionarily related bacteria within the Gram-positives on the basis of the composition of the bases in the DNA. The subclass includes *Streptococcus* spp., *Staphylococcus* spp., *Listeria* spp., *Bacillus* spp., *Clostridium* spp., *Enterococcus* spp. and *Lactobacillus* spp.).

The term *high G+C Gram-positive bacterium* is a term of art defining a particular subclass class of evolutionarily related bacteria within the Gram-positives on the basis of the composition of the bases in the DNA. The subclass includes actinomycetes (actinobacteria) including *Actinomyces* spp., *Arthrobacter* spp., *Corynebacterium* spp., *Frankia* spp., *Micrococcus* spp., *Micromonospora* spp., *Mycobacterium* spp., *Nocardia* spp., *Propionibacterium* spp. and *Streptomyces* spp.

The term *Gram-negative bacterium* is a term of art defining a particular class of bacteria that are grouped together on the basis of certain cell wall staining characteristics. Examples of Gram-negative bacterial genera include *Klebsiella, Acinetobacter, Escherichia, Pseudomonas, Enterobacter* and *Neisseria.*

The term "monodisperse" as applied to the microdroplets means any emulsion showing a coefficient of particle size dispersion, $\varepsilon$, of not more than 1.0, not more than 0.5, and preferably not more than 0.3. Said coefficient $\varepsilon$ is defined by the following equation:

$$\varepsilon = (^{90}D_p - {}^{10}D_p)/^{50}D_p \quad (1)$$

where $^{10}D_p$, $^{50}D_p$ and $^{90}D_p$ are the particle sizes when the cumulative frequencies estimated from a relative cumulative particle size distribution curve for the emulsion are 10%, 50% and 90%, respectively. The case where $\varepsilon=0$ means an ideal state in which emulsion particles show no particle size scattering at all.

As used herein, the term "insertion rate" as applied to transposon insertion, is used to indicate the density of $Tn_A$ insertion at the level of the mutant pool as a whole, with one $Tn_A$ insertion in each bacterium. It will also be understood that lethal $Tn_A$ insertion events, such as those arising from insertional inactivation of an essential gene, will not be represented by viable members of the mutant pool. Thus, the insertion rates specified herein apply to non-essential regions of the DNA.

As used herein, the term "cytotoxic agent" defines any compound (e.g. a metabolite, protein, peptide or other biopolymer) which acts to kill, or to prevent or restrict the growth or biological activity of, a target cell.

Producer prokaryotic cells

Any prokaryotic cell may be used according to the invention, including archaeal and bacterial cells.

Archaeal cells may be selected from the phyla: (a) Crenarchaeota; (b) Euryarchaeota; (c) Korarchaeota; (d) Nanoarchaeota and (e) Thaumarchaeota.

Exemplary, archaeal genera include *Acidianus, Acidilobus, Acidococcus, Aciduliprofundum, Aeropyrum, Archaeoglobus, Bacilloviridae, Caldisphaera, Caldivirga, Caldococus, Cenarchaeum, Desulfurococcus, Ferroglobus, Ferroplasma, Geogemma, Geoglobus, Haladaptaus, Halalkalicoccus, Haloalcalophilium, Haloarcula, Halobacterium, Halobaculum, Halobiforma, Halococcus, Haloferax, Halogeometricum, Halomicrobium, Halopiger, Haloplanus, Haloquadratum, Halorhabdus, Halorubrum, Halosarcina, Halosimplex, Halostagnicola, Haloterrigena, Halovivax, Hyperthermus, Ignicoccus, Ignisphaera, Metallosphaera, Methanimicrococcus, Methanobacterium, Methanobrevibacter, Methanocalculus, Methantxaldococcus, Methanocella, Methanococcoides, Methanococcus, Methanocorpusculum, Methanoculleus, Methanofollis, Methanogenium, Methanohalobium, Methanohalophilus, Methanolacinia, Methanolobus, Methanomethylovorans, Methanomicrobium, Methanoplanus, Methanopyrus, Methanoregula, Methanosaeta, Methanosalsum, Methanosarcina, Methanosphaera, Melthanospirillum, Methanothermobacter, Methanothermococcus, Methanothermus, Methanothrix, Methanotorris, Nanoarchaeum, Natrialba, Natrinema, Natronobacterium, Natronococcus, Natronolimnobius, Natronomonas, Natronorubrum, Nitracopumilus, Palaeococcus, Picrophilus, Pyrobaculum, Pyrococcus,*

*Pyrodictium, Pyrolobus, Staphylothermus, Stetteria, Stygiolobus, Sulfolobus, Sulfophobococcus, Sulfurisphaera, Thermocladium, Thermococcus, Thermodiscus, Thermofilum, Thermoplasma, Thermoproteus, Thermosphaera and Vulcanisaeta.*

Exemplary archaeal species include: *Aeropyrum pernix, Archaeglobus fulgidus, Archaeoglobus fulgidus, Desulforcoccus species TOK, Methanobacterium thermoantorophicum, Methanococcus jannaschii, Pyrobaculum aerophilum, Pyrobaculum calidifontis, Pyrobaculum islandicum, Pyrococcus abyssi, Pyrococcus GB-D, Pyrococcus glycovorans, Pyrococcus horikoshii, Pyrococcus spp. GE23, Pyrococcus spp. ST700, Pyrococcus woesii, Pyrodictium occultum, Sulfolobus acidocaldarium, Sulfolobus solataricus, Sulfolobus tokodalii, Thermococcus aggregans, Thermococcus barossii, Thermococcus celer, Thermococcus fumicolans, Thermococcus gorgonarius, Thermococcus hydrothermalis, Thermococcus onnurineus NA 1, Thermococcus pacificus, Thermococcus profundus, Thermococcus siculi, Thermococcus spp. GE8, Thermococcus spp. JDF-3, Thermococcus spp. TY. Thermococcus thioreducens, Thermococcus zilligti, Thermoplasma acidophilum, Thermoplasma volcanium, Acidianus hospitalis, Acidilobus sacharovorans, Aciduliprofundum boonei, Aeropyrum pernix, Archaeoglobus fulgidus, Archaeoglobus profundus, Archaeoglobus veneficus, Caldivirga maquilingensis, Candidatus Korarchaeum cryptofilum, Candidatus Methanoregula boonei, Candidatus Nitrosoarchaeum limnia, Cenarchaeum symbiosum, Desulfurococcus kamchatkensis, Ferroglobus placidus, Ferroplasma acidarmanus, Halalkalicoccus jeotgali, Haloarcula hispanica, Holaoarcula marismortui, Halobacterium salinarum, Halobacterium species, Halobiforma lucisalsi, Haloferax volvanii, Halogeometricum borinquense, Halomicrobium mukohataei, halophilic archaeon sp. DL31, Halopiger xanaduensis, Haloquadratum walsbyi, Halorhabdus tiamatea, Halorhabdus utahensis, Halorubrum lacusprofundi, Haloterrigena turkmenica, Hyperthermus butylicus, Igniococcus hospitalis, Ignisphaera aggregans, Metallosphaera cuprina, Metallosphaera sedula, Methanobacterium sp. AL-21, Methanobacterium sp. SWAN-1, Methanobacterium thermoautrophicum, Methanobrevibacter ruminantium, Methanobrevibacter smithii, Methanocaldococcus fervens, Methanocaldococcus infernus, Methanocaldococcus jannaschii, Methanocaldococcus sp. FS406-22, Methanocaldococcus vulcanius, Methanocella conradii, Methanocella paludicola, Methanocella sp. Rice Cluster I (RC-I). Methanococcoides burtonii, Methanococcus aeolicus, Methanococcus maripaludis, Methanococcus vannielii, Methanococcus voltae, Methanocorpusculum labreantum, Methanoculleus marisnigri, Methanohalobium evestigatum, Methanohalophilus mahii, Methanoplanus petrolearius, Methanopyrus kandleri, Methanosaeta concilii, Methanosaeta harundinacea, Methanosaeta thermophila, Methanosalsum zhilinae, Methanosarcina acetivorans, Methanosarcina barkeri, Methanosarcina mazei, Methanosphaera stadtmanae, Methanosphaerula palustris, Methanospiriullum hungatei, Mathanothermobacter marburgensis, Methanothermococcus okinawensis, Methanothermus fervidus, Methanotorris igneus, Nanoarchaeum equitans, Natrialba asiatica, Natrialba magadii, Natronomonas pharaonis, Nitrosopumilus maritimus, Picrophilus torridus, Pyrobaculum aerophilum, Pyrobaculum arsenaticum, Pyrobaculum calidifontis, Pyrobaculum islandicum, Pyrobaculum sp. 1860, Pyrococcus abyssi, Pyrococcus furiosus, Pyrococcus horikoshii, Pyrococcus sp. NA42, Pyrococcus yayanosii, Pyrolobus fumarii, Staphylothermus hellenicus, Staphylothermus marinus, Sulfolobus acidocaldirius, Sulfolobus islandicus, Sulfolobus solfataricus, Sulfolobus tokodaii, Thermococcus barophilus, Thermococcus gammatolerans, Thermococcus kodakaraensis, Thermococcus litoralis, Thermococcus onnurineus, Thermococcus sibiricus, Thermococcus sp. 4557, Thermococcus sp. AM4, Thermofilum pendens, Thermoplasma acidophilum, Thermoplasma volcanium, Thermoproteus neutrophilus, Thermoproteus tenax, Thermoproteus uzoniensis, Thermosphaera aggregans, Vulcanisaeta distributa,* and *Vulcanisaeta moutnovskia.*

Particular examples of archaeal cells useful as producer cells according to the invention include *Haloferax volcanii* and *Sulfolobus* spp.

Bacterial cells may be selected from the phylum Actinobacteria, for example from the following families: Actinomycetaceae; Propionibacteriaceae; Frankiaceae; Micrococcaceae; Micromonosporaceae; Streptomycetaceae; Mycobacteriaceae; Corynebacteriaceae; Pseudonocardiaceae and Nocardiaceae.

Thus, in some embodiments, the producer prokaryotic cell is selected from *Saccaropolyspora* spp., for example *Saccaropolyspora erythrea.* In other embodiments, the producer prokaryotic cell is selected from *Kutzneria* spp., for example *Kutzneria albida.* In yet other embodiments, the producer prokaryotic cell is selected from *Mycobacterium* spp., for example *Mycobacterium marinum.*

In preferred embodiments, the producer prokaryotic cell is selected from *Streptomyces* spp. The *Streptomyces* genus comprises more than 500 species, any of which may be used as producer strains according to the invention. Thus, the producer strain may be selected from any of the following species: *S. ambofaciens, S. achromogenes, S. anulatus, S. avermitilis, S. coelicolor, S. clavuligerus, S. felleus, S.ferralitis, S.filamentosus, S. griseus, S. hygroscopicus, S. iysosuperficus, S. lividans, S. noursei, S. scabies, S. somaliensis, S. thermoviolaceus, S. venezuelae* and *S. violaceoruber.*

In some embodiments, the producer prokaryotic cell is selected from: (a) *Streptomyces coelicolor,* (b) *Streptomyces lividans;* (c) *Streptomyces venezuealae;* (d) *Streptomyces griseus;* (e) *Streptomyces avermetilis;* and (f) *Streptomyces bingchenggensis.*

In other embodiments, the producer prokaryotic cell is selected from *Micromonospora* spp., which genus comprises several species, any of which may be used as producer strains according to the invention. Thus, the producer cell may be selected from any of the following species: *M. aurantiaca, M. carbonacea, M. chalcea, M. chersina, M. citrea, M. coerulea, M. echinaurantiaca, M. echinofusca, M. echinospora, M. fulviviridis, M. gallica, M. halophytica, M. inositola, M. inyonensis, M. nigra, M. olivasterospora, M. pallida, M. peucetia, M. purpureochromogenes, M. rosaria, M. sagamiensis* and *M. viridifaciens.*

The bacterial cells may also be selected from the phylum Firmicutes, for example from the following classes: Bacilli, Clostridia and Mollicutes.

Exemplary Bacilli include those selected from any of the following families: Alicyclobacillaceae; Bacillaceae; Caryophanaceae; Listeriaceae; Paenibacillaceae; Planococcaceae; Sporolactobacillaceae; Staphylococcaceae; Thermoactinomycetaceae and Turicibacteraceae; Exemplary Clostridia include those selected from any of the following families: Acidaminococcaceae; Clostridiaceae; Eubacteriaceae; Heliobacteriaceae; Lachnospiraceae; Peptococcaceae; Peptostreptococcaceae and Syntrophomonadaceae.

In some embodiments, the producer prokaryotic cell is selected from *Bacillus* spp. and *Clostridia* spp., for example *Bacillus amyloliquefaciens* subsp. *Plantarum;*

Bacterial cells may also be selected from the family Pseudomonadaceae, for example members of the genus *Pseudomonas.*

Target cells for use in the methods of the invention

*Bacterial target cells*

The target cells for use according to the invention may be bacterial cells. In such embodiments, the bacteria may be selected from: (a) Gram-positive, Gram-negative and/or Gram-variable bacteria; (b) spore-forming bacteria; (c) non-spore forming bacteria; (d) filamentous bacteria; (e) intracellular bacteria; (f) obligate aerobes; (g) obligate anaerobes; (h) facultative anaerobes; (i) microaerophilic bacteria and/or (f) opportunistic bacterial pathogens.

In certain embodiments, target cells for use according to the invention may be selected from bacteria of the following genera: *Acinetobacter (e.g. A. baumannii*); *Aeromonas* (e.g. *A. hydrophila); Bacillus* (e.g. *B. anthracis); Bacteroides* (e.g. *B. fragilis); Bordetella* (e.g. B. *pertussis); Borrelia* (e.g. *B. burgdorferi*); *Brucella* (e.g. *B. abortus, B. canis, B. melitensis* and *B. suis); Burkholderia* (e.g. *B. cepacia* complex); *Campylobacter* (e.g. *C. jejum*); *Chlamydia* (e.g. *C. trachomatis, C. suis* and *C. muridarum); Chlamydophila* (e.g. (e.g. *C. pneumoniae, C. pecorum, C. psittaci, C. abortus, C. felis* and *C. caviae); Citrobacter* (e.g. *C. freundii*); *Clostridium* (e.g. *C. botulinum, C. difficile, C. perfringens* and *C. tetam*); *Corynebacterium* (e.g. *C. diphteriae* and *C. glutamicum); Enterobacter* (e.g. *E. cloacae* and *E. aerogenes); Enterococcus* (e.g. *E. faecalis* and *E. faecium); Escherichia* (e.g. *E. coli*); *Flavobacterium; Francisella* (e.g. *F. tularensis); Fusobacterium* (e.g. *F. necrophorum*); *Haemophilus* (e.g. *H. somnus, H. influenzae* and *H. parainfluenzae); Helicobacter* (e.g. *H. pylori*); *Klebsiella* (e.g. *K. oxytoca* and *K. pneumoniae), Legionella* (e.g. *L. pneumophila);* Leptospira (e.g. *L. interrogans*); *Listeria* (e.g. *L. monocytogenes); Moraxella* (e.g. *M. catarrhalis); Morganella* (e.g. *M. morganii*); *Mycobacterium* (e.g. *M. leprae* and *M. tuberculosis); Mycoplasma* (e.g. *M. pneumoniae); Neisseria* (e.g. *N. gonorrhoeae* and *N. meningitidis*); *Pasteurella* (e.g. *P. multocida); Peptostreptococcus; Prevotella; Proteus* (e.g. *P. mirabilis* and *P. vulgaris), Pseudomonas* (e.g. *P. aeruginosa); Rickettsia* (e.g. *R. rickettsii); Salmonella* (e.g. serotypes . Typhi and Typhimurium); *Serratia* (e.g. *S. marcesens); Shigella* (e.g. S. *flexnaria, S. dysenteriae* and *S. sonnei*); *Staphylococcus* (e.g. *S. aureus, S. haemolyticus, S. intermedius, S. epidermidis* and *S. saprophyticus); Stenotrophomonas* (e.g. *S. maltophila); Streptococcus* (e.g. *S. agalactiae, S. mutans, S. pneumoniae* and *S. pyogenes); Treponema* (e.g. *T. pallidum); Vibrio* (e.g. *V. cholerae) and Yersinia* (e.g. *Y. pestis).*

The target cells for use according to the invention may be selected from high G+C Gram-positive bacteria and in low G+C Gram-positive bacteria.

*Pathogenic bacteria as target cells*

Human or animal bacterial pathogens include such bacteria as *Legionella* spp., *Listeria* spp., *Pseudomonas* spp., *Salmonella* spp., *Klebsiella* spp., *Hafnia* spp, *Haemophilus* spp., *Proteus* spp., *Serratia* spp., *Shigella* spp., *Vibrio* spp., *Bacillus* spp., *Campylobacter* spp., *Yersinia* spp. *Clostridium* spp., *Enterococcus* spp., *Neisseria* spp., *Streptococcus* spp., *Staphylococcus* spp., *Mycobacterium* spp., *Enterobacter* spp.

*Pathogenic fungal cells*

These include yeasts, e.g. *Candida* species including *C. albicans, C krusei* and *C tropicalis,* and filamentous fungi such as *Aspergillus* spp. and *Penicillium* spp. and dermatophytes such as *Trichophyton* spp.

*Plant pathogens*

The target cells for use according to the invention may be plant pathogens, for example *Pseudomonas* spp., *Xylella* spp., *Ralstonia* spp., *Xanthomonas* spp., *Erwinia* spp., *Fusarium* spp., *Phytophthora* spp., *Botrytis* spp., *Leptosphaeria* spp., powdery mildews (Ascomycota) and rusts (Basidiomycota).

Mutant producer cell pools

The methods of the invention involve generating a pool of mutant prokaryotic cells by transposon mutagenesis. The size of the mutant pool affects the resolution of the method: as the pool size increases, more and more different genes with $Tn_A$ insertions will be represented (and so effectively assayed). As the pool size decreases, the resolution of the method reduces, genes will be less effectively assayed, and more and more genes will not be assayed at all.
Ideally, the mutant pool generated in the methods of the invention is *comprehensive,* in the sense that insertions into every (non-essential) gene are represented. The number of $Tn_A$ insertion mutants (i.e. the mutant pool size) required to achieve this depends on various factors, including: (a) the size of the prokaryotic genome; (b) the average size of the genes; and (c) any $Tn_A$ insertion site bias.
With regard to the latter, some areas of the genome attract a low frequency of insertion (especially GC-rich regions). Thus, insertion frequencies and pool sizes large enough to ensure insertions into insertion-refractory regions are preferred.
In general, a minimum insertion rate of one transposon per 25bp is required to achieve a comprehensive pool/library, which typically entails a minimum pool size for prokaryotic cells having a genome size of 4 to 7 Mb of 0.5 x $10^5$ to 1 x $10^5$, for example 5x$10^5$, preferably at least about 1x$10^6$ mutants. In many cases, 1x$10^6$ mutants will allow identification of ~300,000 different insertion sites and correspond to 1 transposon insertion every 13 to 23 bp (or about 40-70 different insertion sites per gene).
However, the methods of the invention do not necessarily require a comprehensive mutant pool (in the sense defined above) in order to generate useful hits. Rather, pool sizes less than the ideal comprehensive pool may be used, provided that a reduction in resolution (and attendant failure to assay certain genes) can be tolerated. This may be the case, for example, where the method is designed to be run iteratively until a hit is identified: in such embodiments the effective pool size grows with each iteration of the method.

Transposon mutagenesis

Transposons, sometimes called transposable elements, are polynucleotides capable of inserting copies of themselves into other polynucleotides. The term transposon is well known to those skilled in the art and includes classes of transposons that can be distinguished on the basis of sequence organisation, for example short inverted repeats at each end; directly repeated long terminal repeats (LTRs) at the ends; and polyA at 3'ends of RNA transcripts with 5' ends often truncated.
Transposomes are transposase-transposon complexes wherein the transposon does not encode transposase. Thus, once inserted the transposon is stable. Preferably, in order to ensure mutant pool stability, the transposon does not encode transposase and is provided in the form of a transposome (i.e. as a complex with transposase enzyme), as described below.
As used herein, the term "activating transposon" (hereinafter abbreviated "$Tn_A$") defines a transposon which comprises a promoter such that transposon insertion increases the transcription of a gene at or near the insertion site. Examples of such transposons are described in Troeschel et al. (2010) Methods Mol Biol. 668:117-39 and Kim et al. (2008) Curr Microbiol. 57(4): 391-394.
The activating transposon/transposome can be introduced into the prokaryotic genome (including chromosomal and/or plasmid DNA) by any of a wide variety of standard procedures which are well-known to those skilled in the art. For example, $Tn_A$ transposomes can be introduced by electroporation (or any other suitable transformation method).
Preferably, the transformation method generates 1 x $10^3$ to 5 x$10^3$ transformants/ng DNA, and such transformation efficiencies are generally achievable using electroporation.
Alternatively, transposon mutagenesis using $Tn_A$ may be performed *in vitro* and recombinant molecules transformed/transfected into the prokaryotic cells. In such embodiments, transposomes can be prepared according to a standard protocol by mixing commercially available transposase enzyme with the transposon DNA fragment. The resulting transposomes are then mixed with extra-chromosomal DNA of interest to allow transposition, then the DNA is introduced into a host bacterial strain using electrotransformation to generate a pool of extra-chromosmal DNA transposon mutants.
In embodiments where mutagenesis is performed *in vitro,* it is possible to mix transposomes with genomic DNA *in vitro* and then introduce the mutagenized DNA (optionally, after fragmentation and/or circularization) into the host prokaryotic cell (e.g. by electroporation) whereupon endogenous recombination machinery incorporates it into the genome. Such an approach may be particularly useful in the case of prokaryotes which are naturally competent (e.g. *Acinetobacter* spp.) and/or can incorporate DNA *via* homologous crossover (e.g. double crossover) recombination events.

Activating transposons for use in the methods of the invention

Any suitable activating transposon may be used in the methods of the invention. Suitable transposons include those based on Tn3 and the Tn3-like (Class II) transposons including $\gamma\delta$ (Tn *1000)*, Tn501, Tn2501, Tn21, Tn917 and their relatives. Also Tn *10,* Tn5, TnphoA, Tn903, TN5096, Tn5099, Tn4556, UC8592, IS493, bacteriophage Mu and related transposable bacteriophages. A variety of suitable transposons are also available commercially, including for example the EZ-Tn5™ < R6Kγori/KAN-2> transposon.

Preferred transposons are those which carry antibiotic resistance genes although any selectable marker can be used including auxotrophic complimentation (which may be useful in identifying mutants which carry a transposon), including Tn5, Tn*10* and TnphoA. For example, Tn*10* carries a tetracycline resistance gene between its IS elements while Tn5 carries genes encoding polypeptides conferring resistance to kanamycin, streptomycin and bleomycin. Other suitable resistance genes include those including neomycin, apramycin, thiostrepton and chloramphenicol acetyltransferase (conferring resistance to chloramphenicol).

It is of course possible to generate new transposons by inserting different combinations of antibiotic resistance genes between IS elements, or by inserting combinations of antibiotic resistance genes between transposon mosaic ends (preferred), or by altering the polynucleotide sequence of the transposon, for example by making a redundant base substitution or any other type of base substitution that does not affect the transposition or the antibiotic resistance characteristics of the transposon, in the coding region of an antibiotic resistance gene or elsewhere in the transposon. Such transposons are included within the scope of the invention.

In many embodiments, a single transposon is used to generate the mutant pool. However, as explained above, the number of Tn insertion mutants (i.e. the mutant pool size) required to achieve a comprehensive pool or library depends *inter alia* on any Tn insertion site bias. Thus, in cases where the transposon insertion site bias occurs, two or more different transposons may be used in order to reduce or eliminate insertion site bias. For example, a combination of two different transposons based on Tn5 and Tn*10* may be employed.

Promoters for use in activating transposons

The nature of the promoter present in the Tn$_A$ is dependent on the nature of the transposon and the ultimate prokaryotic host. Generally, an efficient, outward-oriented promoter which drives constitutive and/or high level transcription of DNA near or adjacent to the insertion site is chosen.

The promoter may include: (a) a Pribnow box (-10 element); (b) a -35 element and/or (c) an UP element.

For example, the *lac* promoter can be used with the EZ-Tn5™ < R6Kγori/KAN-2> transposon, and such constructs are suitable for assay of e.g. *Escherichia coli, Enterobacter* spp. and other members of the family *Enterobacteriaceae* such as *Klebsiella* spp. Other suitable promoters include: *rpl*J (large ribosomal subunit protein; moderate strength promoter); *tac* (artificial *lac*/trp hybrid; strong promoter) and *rrn*B (ribosomal RNA gene promoter; very strong promoter).

Suitable promoters can also be engineered or selected as described in Rhodius et al. (2011) Nucleic Acids Research: 1-18 and Zhao et al. (2013) ACS Synth. Biol. 2 (11): 662-669. The rapid application of next generation sequencing to RNA-seq is now providing a wealth of high-resolution information of transcript start sites at a genomic level, which greatly simplifies the identification of promoter sequences in any given prokaryote. This permits the construction of descriptive promoter models for entire genomes. RNA-seq also provides quantitative information on transcript abundance and hence promoter strength, which enables the construction of promoter strength models that can then be used for predictive promoter strength rankings (see Rhodius et al. (2011) Nucleic Acids Research: 1-18).

Thus, real-time PCR and RNA-seq permit the rapid identification of strong constitutive promoters from the upstream regions of the housekeeping genes in the selected producer cell.

Suitable promoters can also be identified by assay. For example, a series of plasmids can be constructed to test promoter strength empirically. Briefly, the promoter to be tested is placed upstream of an antibiotic resistance gene and then transformed into the relevant bacteria. General cloning assembly and plasmid amplification can be carried out in *E.coli* (facilitated by the ampicillin resistance gene and the pBR322 ori) and the activity of the promoter in the target bacterium can then be assayed by generating a killing curve with the relevant antibiotic - a very high level promoter gives more antibiotic resistance expression and therefore survival at a higher antibiotic concentration. The plasmid series is designed to be modular so that the origin of replication, resistance gene(s) and promoter can be easily switched.

Suitable promoters for use with Actinobacteria in general (and *Streptomyces* spp. in particular) include the actinobacterial *gapdh* and *rpsL* promoters described in Zhao et al. (2013) ACS Synth. Biol. 2 (11): 662-669.

In circumstances where multiple promoters of different strengths are to be used (see below for more details in this regard), then *gapdh* from *Eggerthella lenta* and *rpsL* from *Cellulomonas flavigina* may be used as the basis for very strong promoters, *gapdh* and *rpsL* from *S. griseus* may be used as the basis for medium strength promoters while *rpoA* and *rpoB* from S. *griseus* may be used as the basis for low strength promoters (see Shao et al. (2013) ACS Synth. Biol. 2 (11): 662-669).

Other suitable promoters include *ermE*,* a mutated variant of the promoter of the erythromycin resistance gene from *Saccharopolyspora erythraea* (see e.g. Wagner et al. (2009) J. Biotechnol.142: 200-204).

Suitable promoters for use with Actinobacteria may be identified and/or engineered as described in Seghezzi et al. (2011) Applied Microbiology and Biotechnology 90(2): 615-623, where the use of randomised -10 and -35 boxes to identify important sequences for expression levels is described. Another approach is described in Wang et al. (2013) Applied and Environmental Microbiology 79(14): 4484-4492.

Suitable promoters for use with *Bacillus* spp. May be based on the many different promoters described for the model organism *Bacillus subtilis,* including for example the *P43, amyE* and *aprE* promoters from *B. subtilis* (see e.g. Kim et al. (2008) Biotechnology and Bioprocess Engineering 13(3) 313-318).

Use of multipleTn$_A$/multiple promoters

In some embodiments of the invention, the prokaryotic genome is probed with a mixture of different activating transposons which have outward facing promoters of different strengths. In some circumstances, a broader range of genes involved in antibiotic resistance and/or sensitivity are recovered if a mixture of activating transposons with at least three different promoters of progressively decreasing strength are employed to generate the mutant pool.

In such circumstances, the use of a plurality of activating transposons with promoters of varying strength ensures that transposon insertions occur substantially in all non-essential genes are represented in the initial mutant pool, since transposon insertion can now result in gene activation to yield an appropriate level of transcription (neither too high, nor too low).

In such embodiments, a wide variety of promoters may be used provided that at least three different promoters are used wherein the relative strength of said promoters is: Tn$_A$P1 >Tn$_A$P2 >Tn$_A$P3; such that transposon insertion into prokaryotic DNA generates a pool of mutant cells containing members in which one or more genes are transcribed from Tn$_A$P1, one or more genes are transcribed from Tn$_A$P2 and one or more genes are transcribed from Tn$_A$P3.

Preferably, Tn$_A$P1 is a strong promoter, Tn$_A$P2 a medium-strength promoter and Tn$_A$P3 a weak promoter in the mutagenized prokaryotic host cells under the conditions used for incubation and culture of the mutant pool in the presence of the target cells. In some embodiments, the relative transcription initiation rate of Tn$_A$P1 is at least 3 times, at least 100 times, at least 1000 times or at least 10000 times higher than that of Tn$_A$P3 under these conditions.

Each promoter typically includes: (a) a Pribnow box (-10 element); (b) a -35 element and (c) an UP element. Those skilled in the art are able to readily identify promoters having the required relative strengths by sequence analysis and/or *in vitro* or *in vivo* assays using expression constructs.

Suitable promoters include the *E. coli rpl*J (large ribosomal subunit protein; moderate strength promoter); *tac* (artificial *lac/trp* hybrid; strong promoter) and *rrn*B (ribosomal RNA gene promoter; very strong promoter) promoters.

As used herein, the terms P$_{rplJ}$ and P$_{rrnB}$ specifically refer to the *E. coli* promoters for the 50S ribosomal subunit protein L10 and 16S ribosomal RNA genes, respectively. Orthologues of these (and other) *E. coli* promoters from other Gram-negative bacteria can also be used, including in particular the orthologous *Pseudomonas aeruginosa* or *Acinetobacter baumannii* promoters.

For example, the orthologous *Acinetobacter baumannii* gene corresponding to the E. coli *rrn*B P$_{rrnB}$ has the gene symbol A1S_r12 and encodes the *Acinetobacter baumannii* 16S ribosomal RNA gene, so that the corresponding orthologous promoter is herein designated P$_{(A1S\_r12)}$. Thus, when the method is applied to *Acinetobacter baumannii,* Tn$_{P1}$ may be P$_{(A1S\_r12)}$.

Similarly, when the method of the invention is applied to *Pseudomonas aeruginosa,* Tn$_{P2}$ may be the 16S ribosomal RNA gene promoter from *P. aeruginosa* (i.e. Ps.P$_{rrnB}$) while Tn$_{P3}$ may be selected from the *rps*J (small (30S) ribosomal subunit S10 protein) gene promoter from *P. aeruginosa* (i.e. Ps.P$_{rpsJ}$) and the *E. coli* P$_{rrnB}$.

Effects of Tn$_A$ insertion: generation of mutant producer cells expressing cytotoxic compounds

The use of transposon mutagenesis with an activating transposon (Tn$_A$) according to the methods of the invention is capable of producing an extremely diverse range of phenotypes, since the effect of insertion of the Tn$_A$ can vary from total loss of function, decreased activity, to varying degrees of increased activity, with change of function (and even gain of function) also being possible.

This follows from the fact that the effect of insertion of the Tn$_A$ into the DNA of the prokaryotic producer cell is sequence context dependent. Insertion into the coding sequence of a structural gene may result in insertional inactivation (and so complete loss of function), while insertion upstream of a gene or operon can result in Tn$_A$P driving increased transcription and so lead to expression of that gene or operon (with the extent of overexpression being dependent on subtle positional/polar effects).

A further layer of complexity is introduced by the fact that $Tn_A$ insertion may result in transcripts from both sense and anti-sense strands, resulting in the production of antisense transcripts arising from $Tn_AP$ driving transcription of the non-coding strand of the DNA of the mutant prokaryotic producer cell. Such antisense transcripts may suppress or activate gene expression (for example they may suppress expression of genes by binding to complementary mRNA encoded by the corresponding coding (sense) strand of the DNA of said prokaryotic cell), or may activate gene transcription by suppressing expression of genes encoding gene repressors.

Some of the mechanisms by which transposon mutagenesis with an activating transposon ($Tn_A$) according to the methods of the invention give rise to mutants expressing cytotoxic agents are described in detail below:

## Activation of secondary metabolic processes

Many cytotoxic compounds are the products of secondary metabolic pathways and as such are subject to regulatory mechanisms that serve to maintain the primacy of the primary metabolic pathways required for growth. These mechanisms can limit the production of cytotoxic products to levels which are undetectable and/or inactive in screens based on screens for activity against co-cultured target cells.

The use of transposon mutagenesis with an activating transposon ($Tn_A$) according to the methods of the invention may result in $Tn_A$ insertion into cellular DNA of the producer prokaryotic species which:

(a) occurs up-stream of an activator of a cryptic cytotoxic agent gene or operon, whereat $Tn_AP$ drives increased transcription of the activator which acts *in trans* to increase expression of the cryptic cytotoxic agent gene or operon; or

(b) insertionally inactivates a repressor of a cryptic cytotoxic agent gene or operon, thereby increasing expression of the cryptic cytotoxic agent gene or operon;

In either case, mutants in which existing control of secondary metabolic pathways responsible for the production of cytotoxic agents is effectively overridden are generated.

## Overexpression and inactivation of structural genes

In this context, structural genes are considered to be those coding for any RNA or protein product not associated with regulation.

Structural gene overexpression may increase production of cytotoxic compounds of interest and $Tn_A$ insertion up-stream of an enzyme involved in cytotoxic agent synthesis, whereat $Tn_AP$ drives increased transcription and so leads to increased expression of said enzyme, may therefore result in the generation of an important class of mutants of interest. In a similar fashion, $Tn_A$ insertion up-stream of an enzyme which produces an cytotoxic agent as a side activity, whereat $Tn_AP$ drives increased transcription and so leads to increased expression of said enzyme, thereby increasing its side activity and increasing levels of the cytotoxic agent;

Structural gene inactivation may also increase production of cytotoxic compounds of interest. This is particularly applicable when applied to systems in which one natural product is biosynthetically transformed to another, less useful, compound. $Tn_A$ insertion into cellular DNA of the producer prokaryotic species which insertionally inactivates an enzyme for which an cytotoxic agent is a substrate, so increasing levels of the cytotoxic agent, may therefore result in the generation of mutants of interest.

## Overexpression of export genes

Natural product biosynthesis is often subjected to negative feedback regulation. Clearance from the cell of compounds mediating negative feedback regulation by export proteins or efflux systems can therefore result in the production of cytotoxic compounds of interest. For example, doxorubicin production in *Streptomyces peucetius* can be enhanced over two-fold by overexpression of the export genes *drrA* and *drrB*.

Thus, $Tn_A$ insertion up-stream of an export gene involved in clearance of compounds mediating negative feedback regulation of cytotoxic agent synthesis, whereat $Tn_AP$ drives increased transcription and so leads to increased expression of said export gene, may therefore result in the generation of another important class of mutants of interest.

## Precursor supply

Bottlenecks in biosynthetic pathways can restrict the synthesis of natural products and may be associated with limited provision of key precursors by primary metabolic pathways. Such limitations can be overcome by upregulation of gene or operons that code for enzymes associated with such bottlenecks: increased enzyme levels translate to diminished bottleneck effects and hence improved synthesis of the cytotoxic compounds of interest.

Thus, $Tn_A$ insertion up-stream of an enzyme associated with such bottlenecks in cytotoxic agent synthesis, whereat $Tn_AP$ drives increased transcription and so leads to increased expression of said enzyme, may therefore result in the generation of a yet further class of mutants of interest.

Activation of cryptic genes

Many cytotoxic compounds of interest are the products of secondary metabolic processes which are silent under most growth conditions. Such process may be induced only during particular phases of growth, under certain growth conditions, during particular developmental stages (e.g. sporulation), induction by bacterial cytokines (e.g. as part of a quorum sensing system), stimulation by factors produced by other organisms, nutrient status, temperature, stress or other inter-cellular microbial regulators.

For example, recent genome sequencing projects with *Streptomyces* spp. have revealed that they possess many 'cryptic' antibiotic biosynthetic pathways; that is they have the genetic potential to produce many more antibiotics than previously realised. These cryptic pathways are not genetic relics but can be activated to direct production of new antibiotics.

The use of transposon mutagenesis with an activating transposon ($Tn_A$) according to the methods of the invention may result in $Tn_A$ insertion into cellular DNA of the producer prokaryotic species which:

(a) occurs up-stream of a cryptic cytotoxic agent gene or operon, whereat $Tn_AP$ drives increased transcription and so leads to expression of the cryptic cytotoxic agent gene or operon; or

(b) occurs up-stream of an activator of a cryptic cytotoxic agent gene or operon, whereat $Tn_AP$ drives increased transcription of the activator which acts *in trans* to increase expression of the cryptic cytotoxic agent gene or operon; or

(c) insertionally inactivates a repressor of a cryptic cytotoxic agent gene or operon, thereby increasing expression of the cryptic cytotoxic agent gene or operon.

In each case, mutants in which cryptic metabolic pathways responsible for the production of cytotoxic agents are unveiled are generated.

Functional domain effects

Enzymes involved in the synthesis of cytotoxic agents may be comprised of multiple, functionally distinct, domains. $Tn_A$ insertion into cellular DNA of the producer prokaryotic species which:

(a) insertionally inactivates one or more domains of a multi-domain enzyme, thereby altering its function such that it synthesises, directly or indirectly, an cytotoxic agent; or

(b) occurs up-stream of one or more domains of a multi-domain enzyme, whereat $Tn_AP$ drives increased transcription of said one or more domains, thereby altering the function of said enzyme such that it synthesises, directly or indirectly, an cytotoxic agent;

may therefore result in the generation of a yet further class of mutants of interest.

Finally, the use of transposon mutagenesis with an activating transposon ($Tn_A$) according to the methods of the invention may result in $Tn_A$ insertion into cellular DNA of the producer prokaryotic species which alters the coding repertoire of the prokaryotic producer cell such that an entirely novel cytotoxic agent is directly or indirectly expressed (i.e. the resultant mutation may be neomorphic).

Determining the sequence context of $Tn_A$ insertions

As explained above, the use of transposon mutagenesis with an activating transposon ($Tn_A$) according to the methods of the invention results in the production of a richly diverse mutant pool.

This aspect of the invention is complemented by the ability to establish the sequence context of transposon insertions associated with the production of cytotoxic agents of interest. This greatly facilitates the identification of the cytotoxic agent, the metabolic pathways by which it is synthesised in the cell as well as its mode of action (since the distribution of $Tn_A$ insertions across the entire mutant pool may reveal the identity of resistance factors/mechanisms in co-cultured resistance mutants of the prokaryotic cells).

The sequence context is preferably determined by sequencing DNA adjacent or near (5' and/or 3') the $Tn_A$ insertion site (e.g. by sequencing DNA which comprises $Tn_A$-genomic DNA junctions). Typically, bacterial DNA flanking or adjacent to one or both ends of the $Tn_A$ is sequenced.

The length of adjacent DNA sequenced need not be extensive, and is preferably relatively short (for example, less than 200 base pairs).

Various methods can be used to determine the $Tn_A$ insertion distribution using DNA sequencing: such methods have recently been dubbed Tn-seq procedures (van Opijnen et al. (2009) Nat. Methods 6: 767-772). For example, Tn-seq procedures include affinity purification of amplified Tn junctions (Gawronski et al. (2009) PNAS 106: 16422-16427); ligation of adaptors into genome sequences distal to the end of the transposon using a specialized restriction site (Goodman et al. (2009) Cell Host Microbe 6: 279-289; van Opijnen et al. (2009) Nat. Methods 6: 767-772); selective amplification (Langridge et al. (2009) Genome Research 19: 2308-2316) and the generation of single-stranded DNA circles bearing Tn junctions, which serve as templates for amplification and sequencing after elimination of genomic DNA by exonuclease digestion (Gallagher et al. (2011) mBio 2(1):e00315-10).

Any suitable high-throughput sequencing technique can be used, and there are many commercially available sequencing platforms that are suitable for use in the methods of the invention. Sequencing-by-synthesis (SBS)-based sequencing platforms are particularly suitable for use in the methods of the invention: for example, the Illumina™ system is generates millions of relatively short sequence reads (54, 75 or 100bp) and is particularly preferred.

Other suitable techniques include methods based on reversible dye-terminators. Here, DNA molecules are first attached to primers on a slide and amplified so that local clonal colonies are formed (bridge amplification). Four types of ddNTPs are added, and non-incorporated nucleotides are washed away. Unlike pyrosequencing, the DNA can only be extended one nucleotide at a time. A camera takes images of the fluorescently labelled nucleotides then the dye along with the terminal 3' blocker is chemically removed from the DNA, allowing a next cycle.

Other systems capable of short sequence reads include SOLiD™ and Ion Torrent technologies (both sold by Applied Biosystems™). SOLiD™ technology employs sequencing by ligation. Here, a pool of all possible oligonucleotides of a fixed length are labelled according to the sequenced position. Oligonucleotides are annealed and ligated; the preferential ligation by DNA ligase for matching sequences results in a signal informative of the nucleotide at that position. Before sequencing, the DNA is amplified by emulsion PCR. The resulting bead, each containing only copies of the same DNA molecule, are deposited on a glass slide. The result is sequences of quantities and lengths comparable to Illumina sequencing.

Ion Torrent Systems Inc. have developed a system based on using standard sequencing chemistry, but with a novel, semiconductor based detection system. This method of sequencing is based on the detection of hydrogen ions that are released during the polymerisation of DNA, as opposed to the optical methods used in other sequencing systems. A microwell containing a template DNA strand to be sequenced is flooded with a single type of nucleotide. If the introduced nucleotide is complementary to the leading template nucleotide it is incorporated into the growing complementary strand. This causes the release of a hydrogen ion that triggers a hypersensitive ion sensor, which indicates that a reaction has occurred. If homopolymer repeats are present in the template sequence multiple nucleotides will be incorporated in a single cycle. This leads to a corresponding number of released hydrogens and a proportionally higher electronic signal.

Functional assessment of mutants

Analysis of the sequence information described above may permit an assessment of the functional role of one or more cellular elements in the production and/or mode of action of the cytotoxic agent.

Suitable analytical techniques include bioinformatics, where the (full or partial) sequence of the genetic elements affected by $Tn_A$ insertion is used to interrogate sequence databases containing information from the prokaryotic cell assayed and/or other species in order to identify genes (e.g. orthologous genes in other species) for which essential biochemical function(s) have already been assigned and/or which have been shown to be essential.

Suitable bioinformatics programs are well known to those skilled in the art and include the Basic Local Alignment Search Tool (BLAST) program (Altschul et al. (1990) J. Mol. Biol. 215: 403-410 and Altschul et al. (1997) Nucl. Acids Res. 25: 3389-3402). Suitable databases include, for example, EMBL, GENBANK, TIGR, EBI, SWISS-PROT and trEMBL.

Alternatively, or in addition, the (full or partial) sequence of the implicated genes/genetic elements is used to interrogate a sequence database containing information as to the identity of genes which has been previously constructed using the conventional Tn-seq methods described in the prior art (e.g. as described in Gawronski et al. (2009) PNAS 106: 16422-16427; Goodman et al. (2009) Cell Host Microbe 6: 279-289; van Opijnen et al. (2009) Nat. Methods 6: 767-772; Langridge et al. (2009) Genome Research 19: 2308-2316; Gallagher et al. (2011) mBio 2(1):e00315-10) and/or the techniques described in WO 01/07651.

The position of the inserted promoter can be assessed with respect to its contribution to increased transcription of relevant downstream DNA sequences. A mathematically/technically straightforward bioinformatics component of this technique permits recognition of the contribution of the inserted promoter sequence to transcription of the putative cytotoxic agent gene. Bioinformatics would allow the effects of transcriptional read through on genes downstream of the gene adjacent to the inserted transposon to be considered, where there is there no defined RNA transcription termination sequence.

Microdroplet co-encapsulation

The methods of the invention are suitable for high-throughput screening, since the methods involve compartmentalizing the screening assay in tiny volumes of growth medium in the form of discrete microdroplets. This permits each microdroplet to be treated as a separate culture vessel, permitting rapid screening of large numbers of individual liquid co-cultures using established microfluidic and/or cell-sorting methodologies.

Thus, the microdroplets of the invention function as individual, discrete culture vessels in which the producer and target cells can be co-cultured, analysed, manipulated, isolated and/or sorted.

Any suitable method may be employed for co-encapsulating the mutant producer cells and target cells in microdroplets according to the invention. For example, the mutant producer cells and target cells can be co-encapsulated in gel microdroplets according to the methods described in WO98/41869. Thus, gel droplets can be made using ionic or thermal gelling principles: typically, producer cells and target cells are mixed with a fluid precursor of the gel matrix. This is then solidified by polymerization, causing as little trauma to the cells as possible: for example, shocks arising from changes in temperature, osmotic pressure and pH should be minimized. The use of hydrogels is generally preferred, since they exhibit high water retention and porosity, permitting free diffusion of nutrients and waste products. Many such matrices are known in the art, including polysaccharides, such as alginates, carageenans, agarose, chitosan, cellulose, pectins and polyacrylamides.

Producer and target cells can be added to the gel matrix material to form a suspension and distributed evenly while the matrix is in liquid phase. Gel regions or gel droplets are formed by hardening of the matrix following the formation of small individual volumes of the gel matrix suspension droplets using established techniques. Suitable techniques include, for example, emulsification with oil, vortexing, sonication, homogenization, dropping using a syringe type apparatus, vibration of a nozzle attached to a reservoir of the suspension or atomization followed by electrostatic separation or cutting with rotating wires.

*Emulsion co-encapsulation*

Preferred according to the invention is co-encapsulation by emulsification. As described below, both single and double-phase emulsions may be used, including water-in-oil (W/O) type and water-in-oil-in water (W/O/W) type emulsions. In W/O emulsions, the dispersed phase may comprise microdroplets of aqueous growth medium suspended in a continuous oil phase. In W/O/W emulsions, the dispersed phase may comprise microdroplets of aqueous growth medium enveloped in an oil phase, which microdroplets are in turn suspended in a continuous aqueous phase. Such W/O/W emulsions may exhibit improved rheological properties over simple W/O emulsions: they may for example exhibit lower viscosity, permitting higher flow rates/lower running pressures when sorted using FADS and /or processed using microfluidic devices (see below).

Thus, in the simplest embodiments of the invention, co-encapsulation involves the formation of emulsions comprising microdroplets of liquid growth medium containing mutant producer and target cells dispersed in a carrier liquid as the continuous phase. Such single emulsions are of the W/O type, though it will be appreciated that any suitable liquid may be used as the continuous phase provided that it is immiscible with the growth medium selected for co-culture of the producer and target cells. Typically, however, the carrier liquid is an oil.

Depending *inter alia* on the nature of the continuous phase (e.g. the type of oil) and the droplet size, single emulsions of the type described above may have a viscosity that makes sorting of the microdroplets by certain microfluidic and/or cell sorting techniques difficult. For example, the viscosity may limit the flow rates achievable (e.g. to the range of 10-100 microdroplets per second using FADS - see below) and/or may require undesirably high operating pressures.

*Double emulsions*

Particularly preferred according to the invention are double emulsions of the W/O/W type. Such emulsions comprise microdroplets containing an aqueous core of liquid aqueous growth medium containing mutant producer and target cells enveloped in a shell of immiscible liquid (typically an oil), these microdroplets being dispersed in an aqueous carrier liquid as the continuous phase.

Co-cultures encapsulated in this way can exhibit very low viscosities, and can therefore be sorted using e.g. FADS (see below) at much higher flow rates, permitting sorting of over 10,000 microdroplets per second (see e.g. Bernath et al. (2004) Analytical Biochemistry 325: 151-157).

Co-encapsulation in double emulsions may be achieved by any of a wide variety of techniques, using a wide range of suitable aqueous growth media, carrier oils and surfactants. Suitable techniques for making double emulsions (and for sorting them using FACS) is described, for example, in Bernath et al. (2004) Analytical Biochemistry 325: 151-157.

*Surfactants for use in emulsion co-encapsulation*

As explained herein, the microdroplets (and the corresponding microcultures) of the invention may comprise a single water-in-oil (W/O) or double water-in-oil-in-water (W/O/W) type emulsions, and in such embodiments one or more surfactants may be necessary to stabilize the emulsion.

The surfactant(s) and/or co-surfactant(s) are preferably incorporated into the W/O interface(s), so that in embodiments where single W/O type emulsions are used the surfactant(s) and or co-surfactant(s) may be present in at the interface of the aqueous growth medium microdroplets and the continuous (e.g., oil) phase. Similarly, where double W/O/W type emulsions are used for co-encapsulation according to the invention, the surfactant(s) and or co-surfactant(s) may be present at either or both of the interfaces of the aqueous core and the immiscible (e.g. oil) shell and the interface between the oil shell and the continuous aqueous phase.

A wide range of suitable surfactants are available, and those skilled in the art will be able to select an appropriate surfactant (and co-surfactant, if necessary) according to the selected screening parameters. For example, suitable surfactants are described in Bernath et al. (2004) Analytical Biochemistry 325: 151-157; Holtze and Weitz (2008) Lab Chip 8(10): 1632-1639; and Holtze et al. (2008) Lab Chip. 8(10):1632-1639.

The surfactant(s) are preferably biocompatible. For example, the surfactant(s) may be selected to be non-toxic to the mutant producer and target cells used in the screen). The selected surfactant(s) may also have good solubility for gases, which may be necessary for the growth and/or viability of the encapsulated cells.

Biocompatibility may be determined by any suitable assay, including assays based on tests for compatibility with a reference sensitive biochemical assay (such as *in vitro* translation) which serves as a surrogate for biocompatibility at the cellular level. For example, *in vitro* translation (IVT) of plasmid DNA encoding the enzyme $\beta$-galactosidase with a fluorogenic substrate (fluorescein di-$\beta$-D-galactopyranoside (FDG)) may be used as an indicator of biocompatibility since a fluorescent product is formed when the encapsulated DNA, the molecules involved in transcription and translation, and the translated protein do not adsorb to the drop interface and the higher-order structure of the protein remains intact.

The surfactant(s) may also prevent the adsorption of biomolecules at the microdroplet interface. This may increase the sensitivity of the screen by ensuring that target cells are fully exposed to cytotoxic agents secreted by mutant producer cells. It may also contribute to biocompatibility, e.g. by preventing sequestration of biomolecules necessary for cellular growth, gene expression and/or signalling.

The surfactant may also function to *isolate* the individual microdroplets (and the corresponding microcultures), so that they serve as individual microvessels for co-culture of mutant producer and target cells. In such embodiments, the surfactant may be both hydrophobic and lipophobic, and so exhibit low solubility for the biological reagents of the aqueous phase while inhibiting molecular diffusion between microdroplets.

In some embodiments, the surfactant stabilizes (i.e. prevents coalescence) of a single emulsion comprised of droplets of aqueous media (containing encapsulated cells) dispersed in an oil phase for the duration of the incubation step. Similarly, the surfactant may stabilize droplets of aqueous media (containing encapsulated cells) in a double water-in-oil-in-water (W/O/W) type emulsion for the duration of the incubation step.

Thus, the surfactant may stabilize the microdroplet library under the conditions employed for co-culture of the single mutant producer cell and target cell(s), and so may stabilize the microdroplet at the selected incubation temperature (e.g. about 25°C) for the selected incubation time (e.g. for at least an hour, and in some embodiments for up to 14 days).

Stabilization performance can be monitored by e.g. phase-contrast microscopy, light scattering, focused beam reflectance measurement, centrifugation and/or rheology.

Examples of suitable surfactants include: Abil WE 09 (Evonik - a 1:1:1 mixture of cetyl PEG/PPG 10/1 dimethicone, polyglyceryl-4 isostearate and hexyl laurate); Span® 80; Tween® 20; Tween® 80 and combinations thereof.

*Oils for use in emulsion co-encapsulation*

While it will be appreciated than any liquid immiscible with the aqueous growth medium may be used in the formation of microdroplet emulsions for use according to the invention, the immiscible fluid is typically an oil.

Preferably, an oil is selected having low solubility for biological components of the aqueous phase. Other preferred functional properties include good solubility for gases, the ability to inhibit molecular diffusion between microdroplets and/or combined hydrophobicity and lipophobicity. The oil may be a hydrocarbon oil, but preferred are light mineral oils, fluorocarbon or ester oils. Mixtures of two or more of the above-described oils are also preferred.

Examples of suitable oils include: diethylhexyl carbonate (Tegosoft® DEC (Evonik)); light mineral oil (Fisher); and combinations thereof.

Processes for microdroplet emulsification

A wide range of different emulsification methods are known to those skilled in the art, any of which may be used to create the microdroplets of the invention.

Many emulsification techniques involve mixing two liquids in bulk processes, often using turbulence to enhance drop breakup. Such methods include vortexing, sonication, homogenization or combinations thereof.

In these "top-down" approaches to emulsification, little control over the formation of individual droplets is available, and a broad distribution of microdroplet sizes is typically produced. Alternative "bottom up" approaches operate at the level of individual drops, and may involve the use of microfluidic devices. For example, emulsions can be formed in a microfluidic device by colliding an oil stream and a water stream at a T-shaped junction: the resulting microdroplets vary in size depending on the flow rate in each stream.

A preferred process for producing microdroplets for use according to the invention comprises flow focusing (as described in e.g. Anna et al. (2003) Appl. Phys. Lett. 82(3): 364-366). Here, a continuous phase fluid (focusing or sheath fluid) flanking or surrounding the dispersed phase (focused or core fluid), produces droplet break-off in the vicinity of an orifice through which both fluids are extruded. A flow focusing device consists of a pressure chamber pressurized with a continuous focusing fluid supply. Inside, one or more focused fluids are injected through a capillary feed tube whose extremity opens up in front of a small orifice linking the pressure chamber with the external ambient environment. The focusing fluid stream moulds the fluid meniscus into a cusp giving rise to a steady micro or nano-jet exiting the chamber through the orifice; the jet size is much smaller than the exit orifice. Capillary instability breaks up the steady jet into homogeneous droplets or bubbles.

The feed tube may be composed of two or more concentric needles and different immiscible liquids or gases be injected leading to compound drops. Flow focusing ensures an extremely fast as well as controlled production of up to millions of droplets per second as the jet breaks up.

Other possible microfluidic droplet-forming techniques include pico-injection, whereby oil in water droplets are first formed and then pass down a T junction, along the top of the T and then inner aqueous phase is injected into the oil droplet creating a double emulsion.

In all cases, the performance of the selected microdroplet forming process may be monitored by phase-contrast microscopy, light scattering, focused beam reflectance measurement, centrifugation and/or rheology.

Fluorescence-Activated Droplet Sorting

As explained herein, the methods of invention are suitable for high-throughput screening, since they involve compartmentalizing the screening assay in tiny volumes of growth medium in the form of discrete microdroplets. This permits each microdroplet to be treated as a separate culture vessel, permitting rapid screening of large numbers of individual liquid co-cultures using established microfluidic and/or cell-sorting methodologies.

Thus, following the co-encapsulation step, the resultant microdroplets may be sorted by adapting well-established fluorescence-activated cell sorting (FACS) devices and protocols. This technique has been termed *Fluorescence-Activated Droplet Sorting* (FADS), and is described, for example, in Baret et al. (2009) Lab Chip 9: 1850-1858.

FADS may be used to manipulate the microdroplets at any stage after their formation in the methods of the invention, but are preferably used at least to screen the library of microcultures those in which target cells have been outgrown or overgrown to extinction by mutant producer cells. FADS may also be used during the co-encapsulation step, for example to eliminate empty microdroplets which do not contain mutant producer and/or target cell(s).

Either or both of the mutant producer cells and target cells may be fluorescently labelled to enable FADS. A variety of fluorescent proteins can be used as labels for this purpose, including for example the wild type green fluorescent protein (GFP) of *Aequorea victoria* (Chalfie et al. 1994, Science 263:802-805), and modified GFPs (Heim et al. 1995, Nature 373:663-4; PCT publication WO 96/23810). Alternatively, DNA2.0's IP-Free© synthetic non-Aequorea fluorescent proteins can be used as a source of different fluorescent protein coding sequences that can be amplified by PCR or easily excised using the flanking Bsal restriction sites and cloned into any other expression vector of choice.

Transcription and translation of this type of reporter gene leads to accumulation of the fluorescent protein in the cells, so rendering them amenable to FADS.

Incubation

The incubation conditions and the nature of the aqueous growth medium are selected according to the nature of the selected producer and target cells, and those skilled in the art will be able to readily determine appropriate media, growth temperatures and duration of incubation.

For example, mesophilic organisms will generally be incubated at 15°C - 42°C, while moderate thermophiles will be cultured at higher temperatures (typically 40°C - 60°C).

Thermophiles and hyperthermophiles will be cultured at even higher temperatures (typically 60°C - 80°C and 80°C - 98°C, respectively).

Exemplification

The invention will now be described with reference to specific Examples. These are merely exemplary and for illustrative purposes only: they are not intended to be limiting in any way to the scope of the monopoly claimed or to the invention described. These examples constitute the best mode currently contemplated for practicing the invention.

Example 1: Encapsulation using single emulsion

*Oil mix for continuous phase*

- 73% Tegosoft DEC (Evonik), 20% light mineral oil (Fisher), 7% Abil WE09 (Evonik) (surfactant)
- 70% Tegosoft DEC (Evonik), 20.3% light mineral oil (Fisher) 4.5% Span-80 (surfactant), 4.8% Tween20 (surfactant)
- 90% light mineral oil, 10% Span-80 (surfactant)

All oil mixes need to be made at least 30 minutes prior to use but can be kept indefinitely.

*Aqueous growth media for dispersed phase*

This may be selected from the following:

- SOC broth (20g/L tryptone, 5g/L yeast extract 10mM NaCl, 2.5mM KCl, 10mM $MgCl_2$, 10mM $MgSO_4$ and 20mM glucose)
- SOC + 5% Glycerol
- LB broth (10g/L Peptone, 5g/L Yeast extract, 10g/L NaCl)
- 2 x YT (16g/L tryptone, 10g/L Yeast Extract, 5g/NaCl)
- 2 x YT + 0.5% Glucose and 5% Gylcerol
- 2 x YT + 5% glycerol
- Peptone glycerol media 5g/L peptone, 5% glycerol

The inclusion of glycerol as a carbon source can facilitate microdroplet formation by vortexing.

1. 0.5ml to 10ml of growth media containing the producer and target cells at appropriate cell numbers to give <1 producer and then ≥1 target cell per droplet produced. This is aliquoted into a suitable vessel (Eppendorf tube 1.5 or 2ml 15ml or 50ml falcon or 24 well plate are acceptable).

2. This growth media is then overlaid with 1-2x volume of the oil mix.

3. This is the vortexed for between 3-6 minutes (usually 5minutes) at 12,000-18,000 rpm. The speed varies by oil and media used as well as the desired droplet size as a general rule the higher the speed and the longer the vortexing the smaller the average droplet size (although a range of sizes are always generated by this method).

4. The integrity of the droplets and presence of cells within is screened visually under a microscope using phase contrast. Alternative visualisation methods can be used e.g. fluorescence.

5. Droplets are then incubated at appropriate temperature (usually 37°C, but they are stable between 4°C and 95°C) for time periods of 1h to ≥14 days.

6. Droplets can then be sorted by FADS if required and then desired cells recovered from the droplets.

7. To break open droplets additional surfactant is added (e.g. 1% SDS for Tegosoft oil, but any appropriate surfactant such as Tweens, Sarcosyl etc. can be used) this disrupts droplet integrity releasing cells.

8. Some oil mixes can be lysed by freezing the solution disrupting the droplets.

9. Cells can then be recovered by centrifugation, or the sample can be applied directly to columns for DNA extractions.

Example 2: Encapsulation using double emulsion

*Aqueous growth media*

As in Example 1 (above).

*Mixes used to produce outer aqueous phase*

• Phosphate buffered Saline (PBS; 10mM phosphate buffer, 137 mM NaCl) with 2% Tween-20

• PBS with 2% Tween-80 (surfactant)

*Oil mix for droplet shells*

As for the oil mixes set out in in Example 1 (above).

1. 0.5ml to 10ml of growth media containing the producer and target cells at appropriate cell numbers to give <1 producer and then ≥1 target cell per droplet produced. This is aliquoted into a suitable vessel (Eppendorf tube 1.5 or 2ml 15ml or 50ml falcon or 24 well plate are acceptable).

2. This growth media is then overlaid with 1-2x volume of the oil mix.

3. This is the vortexed for between 3-6 minutes (usually 5minutes) at 12,000-18,000 rpm. The speed varies by oil and media used as well as the desired droplet size as a general rule the higher the speed and the longer the vortexing the smaller the average droplet size although a range of sizes are always generated by this method.

4. The integrity of the droplets and presence of cells within is screened visually under a microscope using phase contrast. Alternative visualisation methods can be used e.g. fluorescence.

5. A second vortexing step is then introduced to generate the double emulsion. A volume equal to the oil volume of second aqueous phase with surfactant is added to the single emulsion. This is then vortexed as but at reduce rpm (6,000 to 10,000 rpm) for just 2-3 minutes. The formation of double emulsion droplets can then be visualised on a microscope as before.

6. Droplets are then incubated at appropriate temperature (usually 37°C, but they are stable between 4°C and 95°C) for time periods of 1h to ≥14 days.

7. Droplets can then be sorted by FADS if required and then desired cells recovered from the droplets.

8. To break open droplets additional surfactant is added (e.g. 1% SDS for Tegosoft oil, but any appropriate surfactant such as Tweens, Sarcosyl or similar can be used) this disrupts droplet integrity releasing cells.

9. Some oil mixes can be lysed by freezing the solution disrupting the droplets.

10. Cells can then be recovered by centrifugation, or the sample can be applied directly to columns for DNA extractions.

Example 3: Production of microdroplets using a microfluidic chip

Droplet generation in a microfluidic allows creation of single and double emulsions. In its simplest form double emulsions are formed by sequential formation of and oil in water droplet and then formation of a second aqueous layer by the same methodology.
Alternative methods involve simultaneous co-encapsulation of aqueous phase in oil and then the oil-aqueous droplet in a secondary continuous phase of aqueous media. Droplet formation on a chip leads to generation of highly uniform sized droplets. Size is directly related to the size of the channels on the fluidic and the flow rates used to generate droplets. Microfluidic droplet formation allows the use of novel oil and surfactant mixes not available when producing droplets in bulk "top-down" approaches.

The producer and target cells in suitable growth media are mixed at appropriate ratios to allow for ≤1 producer per droplet and then ≥1 target cell per droplet. This aqueous mixture of cells is then pumped through a microfluidic device with geometry that allows the formation of water in oil single emulsion droplets, or double emulsion droplets, as detailed below.

These droplets are collected in a vessel suitable for incubation and the incubated at appropriate temperature (usually 37°C, but they are stable between 4°C and 95°C) for time periods of 1h to ≥14 days.

Droplets can then be sorted by FADS if required and then desired cells recovered from the droplets.

To break open droplets, additional surfactant is added (e.g. 1% SDS for Tegosoft oil, but any appropriate surfactant such as Tweens, Sarcosyl etc. can be used) to disrupt droplet integrity, so releasing the cells. Some oil mixes can be lysed by freezing the solution disrupting the droplets.

Cells can then be recovered by centrifugation, or the sample can be applied directly to columns for DNA extractions.

Example 4: Production of double emulsion microdroplets using flow focusing

Here, oil flows from the sides into a hydrophobic channel causing pinch off of the aqueous phase generating the water in oil droplets. This method allows good size control and 1000's of droplets per second to be generated depending on flow rates of the second liquid phases.

It is possible to use two such chips connected in series to generate double emulsions: the first droplets are aqueous phase in oil which are pushed through a second chip as if aqueous phase, the side flowing oil being replaced with the secondary aqueous phase.

It is also possible to integrate both steps into a single chip and junction interface; whereby water in oil droplets are formed and immediately surrounded in the outer aqueous phase to generate the W/O/W double emulsion.

The hydrophilic and hydrophobic coatings are inverted on the second chip, thus the second chip has the same geometry, but the opposite surface coatings to facilitate flow of aqueous phase as the carrier/sheath fluid. This permits a two stage encapsulation: the first to generate single phase emulsions which are used for incubation and co-culturing of the producer and target cells, and a second stage in which the microcultures are converted into double emulsions for subsequent FADS.

Example 5: Co-culture conditions for prokaryotic and eukaryotic cells

Balanced co-culture of bacterial and eukaryotic cells (i.e. culture conditions under which the doubling rates of both prokaryotic and eukaryotic cells are not so different as to result in rapid overgrowth of one class of cells) can be readily achieved by selection of appropriate culture media and incubation conditions (including *inter alia* the extent of aeration and temperature of incubation).

Table 1 below shows the growth of soil bacteria used for co-culture in commercially available tissue culture media and under conditions identical to those used to culture mammalian cells in commonly used mammalian culture media (i.e. at 37°C). Table 2 shows the published growth rates of a selection of eukaryotic cell-lines under these conditions. Table 3 shows the growth of soil bacteria used for co-culture in commercially available tissue culture media at 30°C (rather than 37°C), but otherwise under conditions identical to those used to obtain the data shown in Table 1.

Table 1: Growth at 37°C

| Strain Media | *Pseudomonas flourescens* | *Pseudomonas putida* | *Pseudomonas mandelii* | *Streptomyces venezuelae* | *Streptomyces 14100* |
|---|---|---|---|---|---|
| Freestyle | 43.6 hours | 45.8 hours | 51.1 hours | 33.07 hours | 22.7 hours |
| DMEM + 10% FBS | 34.9 hours | 48.7 hours | 34.8 hours | 27.0 hours | 29.1 hours |
| RPMI + 10% FBS | 33.0 hours | 32.75 hours | 35.2 hours | 38.8 hours | 29.1 hours |

Table 2: Representative growth rates of eukaryotic cells

| Cell lines | HEK-293 | COS-7 | Jurkat | Hybridoma | Human A549 | Murine 264 cell line | B16F10 |
|---|---|---|---|---|---|---|---|
| Doubling time (h) | 10 | 18 | 48 | 14 | 27 | 11 | 12 |

Table 3: Growth at 30°C

| Strain Media | *Pseudomonas flourescens* | *Pseudomonas putida* | *Pseudomonas mandelii* | *Streptomyces venezuelae* | *Streptomyces 14100* |
|---|---|---|---|---|---|
| Freestyle | 15.7 hours | 21.0 hours | 18.5 hours | 22.4 hours | 13.3 hours |
| DMEM | 19.5 hours | 19.3 hours | 23.0 hours | 18.7 hours | 16.1 hours |
| RPMI | 23.9 hours | 22.5 hours | 21.1 hours | 52.0 hours | 20.4 hours |

Growth rate was calculated by growing fresh overnight cultures of each culture at 30°C in the desired mammalian culture media. This was then diluted 1 in 50 into fresh tissue culture media and the $OD_{600}$ then measured at various time points during growth at 37°C + 5% $CO_2$ or at 30°C. The growth rate was calculated from the doubling time during exponential growth of the culture, and based on individual cultures grown in triplicate.

As can be seen from the above data, the relative growth rates of prokaryotic and eukaryotic cells in co-culture can be readily controlled to achieve balanced co-cultures by *inter alia* the selection of appropriate culture conditions, including incubation temperature.

**Claims**

1. A method for screening mutant prokaryotic cells to identify producers of a cytotoxic agent active against a target cell, the method comprising the steps of:

   (a) providing cells of a producer prokaryotic species;
   (b) generating a pool of mutant producer cells by transposon mutagenesis of the cells of step (a) with an activating transposon ($Tn_A$), wherein the $Tn_A$ comprises an outward-facing promoter ($Tn_AP$) capable of increasing transcription of a gene at or near its insertion site in the DNA of said producer cells;
   (c) co-encapsulating individual members of the pool of step (b) with one or more target cells in microdroplets, the microdroplets comprising a volume of aqueous growth media suspended in an immiscible carrier liquid, thereby generating a library of microdroplets each comprising a single mutant producer cell and one or more target cell(s);
   (d) incubating the microdroplet library of step (c) under conditions suitable for co-culture of the single mutant producer cell and target cell(s) to produce a library of microcultures, whereby mutant producer cells producing a cytotoxic agent active against the target cell(s) outgrow target cells in each microculture; and
   (e) screening the library of microcultures of step (d) for microcultures in which target cells have been outgrown or overgrown to extinction by mutant producer cells.

2. The method of claim 1 further comprising the step of sequencing the DNA of mutant producer cells in microdroplets in which target cells have been outgrown or overgrown to extinction by mutant producer cells, optionally wherein: (i) DNA adjacent or near the insertion site of the TnA is sequenced, for example by sequencing which comprises selective amplification of transposon-cellular DNA junctions; and/or (ii) the sequencing comprises high-throughput massively parallel sequencing, for example selected from: (a) sequencing-by-synthesis (SBS) biochemistry; and/or (b) nanopore sequencing; and/or (c) tunnelling current sequencing; and/or (d) pyrosequencing; and/or (e) sequencing-by-ligation (SOLiD sequencing); and/or (f) ion semiconductor; and/or (g) mass spectrometry sequencing; and/or (iii) about 25, 50, 75, 100 or greater than 100 base pairs of DNA adjacent or near the TnA insertion site are sequenced; and/or (iv) the sequenced DNA is 5' and/or 3' to the TnA insertion site.

3. The method of claim 1 or claim 2 further comprising the step of sequencing mRNA transcripts produced by TnAP in mutant producer cells in microdroplets in which target cells have been outgrown or overgrown to extinction by mutant producer cells to produce an mRNA transcript profile, optionally wherein said mRNA transcript profile comprises a determination of:

(a) the sequences of said mRNA transcripts produced by $Tn_AP$; and/or

(b) the start and finish of mRNA transcripts produced by TnAP; and/or

(c) the lengths of said mRNA transcripts produced by $Tn_AP$; and/or

(d) the relative abundance of said mRNA transcripts produced by $Tn_AP$; and/or

(e) the site of transcription on the cellular DNA; and/or

(f) whether the mRNA transcripts produced by $Tn_AP$ is sense or antisense with respect to the cellular DNA; and/or

(g) whether the mRNA transcripts produced by $Tn_AP$ correspond to ORFs with respect to the cellular DNA; and/or

(h) whether the mRNA transcripts produced by $Tn_AP$ encode prokaryotic proteins and/or protein domains.

4. The method of any one of the preceding claims wherein the microdroplets: (a) are substantially spherical and have a diameter of: (a) 10 μm to 500 μm; (b) 10 μm to 200 μm; (c) 10 μm to 150 μm; (d) 10 μm to 100 μm; (e) 10 μm to 50 μm; or (f) about 100 μm; and/or (b) comprise a volume of aqueous growth media in the gel or liquid state; and/or (c) comprise an inner core of aqueous growth media enveloped in an outer oil shell, the carrier liquid being a continuous aqueous phase, optionally wherein the inner aqueous core has a diameter of: (a) 10 μm to 500 μm; (b) 10 μm to 200 μm; (c) 10 μm to 150 μm; (d) 10 μm to 100 μm; (e) 10 μm to 50 μm; or (f) about 100 μm and/or wherein the outer oil shell has a thickness of: (a) 10 μm to 200 μm; (b) 10 μm to 200 μm; (c) 10 μm to 150 μm; (d) 10 μm to 100 μm; (e) 10 μm to 50 μm; or (f) about 100 μm.

5. The method of any one claims 1-4 wherein the carrier liquid is a water-immiscible liquid, optionally wherein: (i) the water-immiscible liquid is an oil, for example selected from: (a) a hydrocarbon oil; (b) a fluorocarbon oil; (c) an ester oil; (d) an oil having low solubility for biological components of the aqueous phase; (e) an oil which inhibits molecular diffusion between microdroplets; (f) an oil which is hydrophobic and lipophobic; (g) an oil having good solubility for gases; and/or (h) combinations of any two or more of the foregoing; and/or (ii) the microdroplets are comprised in a W/O emulsion wherein the microdroplets constitute an aqueous, dispersed, phase and the carrier liquid constitutes a continuous oil phase.

6. The method of any one of claims 1-4 wherein the carrier liquid is an aqueous liquid, for example phosphate buffered saline (PBS), and the microdroplets are optionally comprised in a W/O/W double emulsion wherein the microdroplets comprise: (a) an inner core of aqueous growth media enveloped in an outer oil shell as the dispersed phase, and (b) the carrier liquid as the continuous aqueous phase.

7. The method of any one of the preceding claims wherein the microdroplets are comprised in an emulsion, wherein the carrier liquid constitutes the continuous phase and the microdroplets constitute the dispersed phase, and wherein the emulsion further comprises a surfactant and optionally a co-surfactant, optionally wherein: (a) the surfactant and/or co-surfactant is located at the interface of the dispersed and continuous phases; and/or (b) the microdroplets are comprised in a W/O/W double emulsion as defined in claim 6 and the surfactant and/or co-surfactant is located at the interface of aqueous core and oil shell; and/or (c) the surfactant and/or co-surfactant comprises a fluorosurfactant having a non-ionic headgroup; and/or (d) the surfactant and/or co-surfactant comprises a perfluorinated polyether (PFPE); and/or (e) the surfactant and/or co-surfactant comprises a PFPE-polyethylene glycol (PEG) block-copolymer, for example a diblock or triblock copolymer, for example a PFPE-PEG-PFPE triblock copolymer.

8. The method of any one of the preceding claims wherein the microdroplets are monodisperse.

9. The method of any one of the preceding claims wherein:

(A) the co-encapsulation step (c) comprises mixing: (a) the pool of mutant producer cells; (b) a population of the target cells; (c) an aqueous growth medium; (d) a water-immiscible liquid, for example an oil as defined in claim 5; and (e) a surfactant, for example as defined in claim 7, under conditions whereby a W/O type single emulsion comprising microdroplets of the aqueous growth medium dispersed in the water-immiscible liquid is formed, optionally wherein following the incubation step (d), an aqueous carrier liquid, for example as defined in claim 6, is mixed with the single emulsion of step (c) under conditions whereby a W/O/W double emulsion comprising microdroplets of the aqueous growth medium enveloped in the water-immiscible liquid and dispersed in the aqueous carrier liquid is formed; or

(B) the co-encapsulation step (c) comprises mixing: (a) the pool of mutant producer cells; (b) a population of the target cells; (c) an aqueous growth medium; (d) a water-immiscible liquid, for example an oil as defined in claim 5; (e) a surfactant, for example as defined in claim 7, and (f) an aqueous carrier liquid, for example as defined in claim 6, under conditions whereby a W/O/W double emulsion comprising microdroplets of the aqueous growth medium enveloped in the water-immiscible liquid and dispersed in the aqueous carrier liquid is formed;

optionally wherein the mixing comprises: (a) vortexing and/or (b) sonication; (c) homogenization; (d) pico-injection and/or (e) flow focusing and/or the co-encapsulation step (c) further comprises eliminating empty microdroplets which do not contain mutant producer and/or target cell(s), for example wherein the mutant producer cells and/or target cells are fluorescently labelled and empty microdroplets are eliminated by FADS.

10. The method of any one of the preceding claims wherein incubation step (d) comprises maintaining the microdroplet library at a temperature of 15°C - 95°C for at least 1 hour, optionally wherein: (a) the microdroplet library is maintained at a temperature of: (i) 15°C - 42°C; (ii) 20°C - 40°C; (iii) 20°C - 37°C; (iv) 20°C - 30°C; or (v) about 25°C; (vi) 40°C - 60°C; (vii) 60°C - 80°C; or (viii) 80°C - 98°C; (b) the incubation step (d) comprises maintaining the microdroplet library at said temperature for about 2, 4, 6, 12, 24 or 48 hours or for up to 7 days, for example for 1, 2, 3, 4, 5, 6 or 7 days; (c) the incubation step (d) comprises maintaining the microdroplet library at said temperature for up to 2 weeks, for example for 1 week or 2 weeks.

11. The method of any one of the preceding claims wherein:

   (A) the screening step (e) comprises: (i) eliminating microdroplets which contain target cells, optionally wherein the target cells are fluorescently labelled and microdroplets which contain target cells are eliminated by FADS; and/or (ii) comprises FADS sorting for microdroplets in which target cells have been outgrown or overgrown to extinction by mutant producer cells; and/or
   (B) the method further comprises a potency analysis step carried out during incubation step (d) whereby the relative growth rate in co-culture of mutant producer cells and target cells is determined, optionally wherein: (i) the potency analysis step comprises selection of microdroplets which contain only mutant producer cells by FADS, for example wherein two or more successive rounds of FADS selection are carried out at during incubation; and/or (ii) target cells are fluorescently labelled and microdroplets which contain target cells are subjected to continued incubation.

12. The method of any one of the preceding claims wherein:

   (A) the producer prokaryotic species is selected from:

   archaea, for example selected from the phyla: (a) Crenarchaeota; (b) Euryarchaeota; (c) Korarchaeota; (d) Nanoarchaeota and (e) Thaumarchaeota, for example *Haloferax volcanii* or *Sulfolobus* spp.; and/or
   bacteria, for example selected from: (a) actinomycetes; (b) *Pseudomonas* spp., and (c) *Bacillus* spp., optionally selected from *Streptomyces* spp, for example *Streptomyces coelicolor; Streptomyces lividans; Streptomyces venezuealae; Streptomyces griseus; Streptomyces avermetilis; Streptomyces bingchenggensis; Kutzneria albida; Saccaropolyspora erythrea; Mycobacterium marinum; Bacillus amyloliquefaciens* subsp. *Plantarum;* and *Micromonospora echinospora.*

   and/or
   (B) the target cell is a bacterial or eukaryotic cell, optionally being fungal; mammalian; a higher plant cell; protozoal; a helminth cell; algal; or an invertebrate cell, for example a cancer cell, for example a human cancer cell, or a pathogenic bacterium, for example selected from: *Pseudomonas aeruginosa, Acinetobacter baumannii, Escherichia coli, Klebsiella pneumoniae, Staphylococcus aureus, Enterococcus faecium, Enterococcus faecalis* and *Enterobacter* spp.

13. The method of any one of the preceding claims wherein:

   (A) the mutant producer cell and/or target cell(s) of step (c) are labelled, e.g. with a fluorescent label and/or wherein the screening step (e) comprises FADS; and/or
   (B) the pool of mutant producer cells comprises: (a) at least $0.5 \times 10^5$ mutants, for example at least $1 \times 10^5$ mutants; (b) at least $5 \times 10^5$ mutants; (c) at least $1 \times 10^6$ mutants; (d) $0.5 \times 10^6$ to $2 \times 10^6$ mutants; (e) about $1 \times 10^6$ mutants; or (f) up to $10 \times 10^6$ mutants; and/or
   (C) the transposon mutagenesis step (b) yields an insertion rate of: (a) at least one transposon per 50 base pairs of bacterial DNA; (b) at least one transposon per 30 base pairs of bacterial DNA; (c) at least one transposon per 25 base pairs of bacterial DNA; (d) at least one transposon per 15 base pairs of bacterial DNA; (e) at least one transposon per 10 base pairs of bacterial DNA; or (f) at least one transposon per 5 base pairs of bacteria DNA; and/or
   (D) the transposon mutagenesis step (b) comprises insertion of the $Tn_A$ into: (a) chromosomal (genomic) DNA;

(b) plasmid DNA, or (c) a mixture of chromosomal (genomic) and plasmid DNA of the cells of the producer bacterial species; and/or

(E) the transposon mutagenesis of step (b) occurs *in vivo* or *in vitro;* and/or

(F) the cytotoxic agent is selected from: (a) an antibiotic; (b) an antineoplastic agent; and (c) an antimycotic agent; and/or

(G) in step (b) $Tn_A$ insertion into cellular DNA of the producer prokaryotic species:

> (a) occurs up-stream of a cryptic cytotoxic agent gene or operon, whereat $Tn_AP$ drives increased transcription and so leads to expression of the cryptic cytotoxic agent gene or operon;
> (b) occurs up-stream of an activator of a cryptic cytotoxic agent gene or operon, whereat $Tn_AP$ drives increased transcription of the activator which acts *in trans* to increase expression of the cryptic cytotoxic agent gene or operon;
> (c) insertionally inactivates a repressor of a cryptic cytotoxic agent gene or operon, thereby increasing expression of the cryptic cytotoxic agent gene or operon;
> (d) occurs up-stream of an enzyme involved in cytotoxic agent synthesis, whereat $Tn_AP$ drives increased transcription and so leads to increased expression of said enzyme;
> (e) insertionally inactivates an enzyme for which an cytotoxic agent is a substrate, so increasing levels of the cytotoxic agent;
> (f) occurs up-stream of an enzyme which produces an cytotoxic agent as a side activity, whereat $Tn_AP$ drives increased transcription and so leads to increased expression of said enzyme, thereby increasing its side activity and increasing levels of the cytotoxic agent;
> (g) insertionally inactivates one or more domains of a multi-domain enzyme, thereby altering its function such that it synthesises, directly or indirectly, an cytotoxic agent;
> (h) occurs up-stream of one or more domains of a multi-domain enzyme, whereat $Tn_AP$ drives increased transcription of said one or more domains, thereby altering the function of said enzyme such that it synthesises, directly or indirectly, an cytotoxic agent; or
> (i) alters coding repertoire such that an cytotoxic agent is directly or indirectly expressed.

14. A method of identifying a cytotoxic agent comprising screening mutant bacteria to identify producers of a cytotoxic agent active against a target cell according to a method as defined in any one of the preceding claims.

15. A process for producing a cytotoxic agent comprising the method as defined in claim 14, further comprising synthesising or isolating said cytotoxic agent from the mutant bacteria, and then optionally mixing the synthesised or isolated cytotoxic agent with a pharmaceutically acceptable excipient to produce a pharmaceutical composition.

**Patentansprüche**

1. Verfahren zum Screening von mutierten prokaryotischen Zellen, um Produzenten eines zytotoxischen Mittels, das gegen eine Zielzelle aktiv ist, zu identifizieren, wobei das Verfahren die folgenden Schritte umfasst:

(a) Bereitstellen von Zellen einer prokaryotischen Produzentenspezies;
(b) Erzeugen eines Pools von mutierten Produzentenzellen durch eine Transposon-Mutagenese der Zelle aus Schritt (a) mit einem aktivierenden Transposon ($Tn_A$), wobei das $Tn_A$ einen nach außen gerichteten Promotor ($Tn_AP$) aufweist, der dazu fähig ist, die Transkription eines Genes bei oder in der Nähe seiner Einfügungsstelle in der DNA der Produzentenzellen zu erhöhen;
(c) gemeinsames Einkapseln von individuellen Mitgliedern des Pools aus Schritt (b) mit einer oder mehreren Zielzellen in Mikrotröpfchen, wobei die Mikrotröpfchen ein Volumen eines wässrigen Wachstumsmediums umfassen, das in einer nicht mischbaren Trägerflüssigkeit suspendiert ist, wodurch eine Bibliothek von Mikrotröpfchen erzeugt wird, von denen jedes eine einzelne mutierte Produzentenzelle und eine oder mehrere Zielzelle(n) umfasst;
(d) Inkubieren der Mikrotröpfchen-Bibliothek aus Schritt (c) unter Bedingungen, die zum gemeinsamen Kultivieren der einzelnen mutierten Produzentenzelle und der Zielzelle(n) geeignet sind, um eine Bibliothek von Mikrokulturen zu erzeugen, wobei mutierte Produzentenzellen, die ein zytotoxisches Mittel produzieren, das aktiv gegen die Zielzelle(n) ist, in jeder Mikrokultur über die Zielzellen hinauswachsen; und
(e) Screening der Bibliothek von Mikrokulturen aus Schritt (d) nach Mikrokulturen, in denen mutierte Produzentenzellen über die Zielzellen hinausgewachsen sind oder diese bis zur Extinktion überwuchert haben.

2. Verfahren nach Anspruch 1, ferner umfassend den Schritt des Sequenzierens der DNA von mutierten Produzentenzellen in Mikrotröpfchen, in denen mutierte Zellen über die Zielzellen hinausgewachsen sind oder diese bis zur Extinktion überwuchert haben, gegebenenfalls wobei: (i) DNA, die der Einfügungsstelle des TnA benachbart oder in deren Nähe ist, sequenziert wird, zum Beispiel durch ein Sequenzieren, das eine selektive Verstärkung von transposonzellulären DNA-Verbindungen umfasst; und/oder (ii) das Sequenzieren ein massives paralleles Hochdurchsatzsequenzieren umfasst, zum Beispiel ausgewählt aus: (a) einer Sequenzieren-durch-Synthese-(SBS)-Biochemie; und/oder (b) einem Nanoporensequenzieren; und/oder (c) einem Tunnelstromsequenzieren; und/oder (d) einem Pyrosequenzieren; und/oder (e) einem Sequenzieren-durch-Ligation (SOLiD-Sequenzieren); und/oder (f) einem Ionenhalbleiter; und/oder (g) einem Massenspektrometriesequenzieren; und/oder (iii) etwa 25, 50, 75, 100 oder mehr als 100 Basenpaare von DNA, die der TnA-Einfügungsstelle benachbart oder in deren Nähe sind, sequenziert werden; und/oder (iv) die sequenzierte DNA 5' und/oder 3' zu der TnA-Einfügungsstelle ist.

3. Verfahren nach Anspruch 1 oder Anspruch 2, ferner umfassend den Schritt des Sequenzierens von mRNA-Transkripten, die durch den TnAP von mutierten Produzentenzellen in Mikrotröpfchen produziert werden, in denen mutierte Produzentenzellen über Zielzellen hinausgewachsen sind oder diese bis zur Extinktion überwuchert haben, um ein mRNA-Transkript-Profil zu produzieren, wobei das mRNA-Transkript-Profil gegebenenfalls eine Bestimmung von Folgendem umfasst:

(a) der Sequenzen der mRNA-Transkripte, die durch den $Tn_AP$ produziert werden; und/oder

(b) des Beginns und des Endes von mRNA-Transkripten, die durch den TnAP produziert werden; und/oder

(c) der Länge der mRNA-Transkripte, die durch den $Tn_AP$ produziert werden; und/oder

(d) des relativen Überflusses von mRNA-Transkripten, die durch den $Tn_AP$ produziert werden; und/oder

(e) der Stelle der Transkription auf der zellulären DNA; und/oder

(f) ob die mRNA-Transkripte, die durch den $Tn_AP$ produziert werden, Sense- oder Antisense-Stränge hinsichtlich der zellulären DNA sind; und/oder

(g) ob die mRNA-Transkripte, die durch den $Tn_AP$ produziert werden, OLR hinsichtlich der zellulären DNA entsprechen; und/oder

(h) ob die mRNA-Transkripte, die durch den $Tn_AP$ produziert werden, prokaryotische Proteine und/oder Proteindomänen codieren.

4. Verfahren nach einem der vorhergehenden Ansprüche, wobei die Mikrotröpfchen: (a) im Wesentlichen kugelförmig sind und einen Durchmesser aufweisen von: (a) 10 μm bis 500 μm; (b) 10 μm bis 200 μm; (c) 10 μm bis 150 μm; (d) 10 μm bis 100 μm; (e) 10 μm bis 50 μm; oder (f) etwa 100 μm; und/oder (b) ein Volumen eines wässrigen Wachstumsmediums im Gel- oder Flüssigzustand umfassen; und/oder (c) einen inneren Kern aus einem wässrigen Wachstumsmedium umfassen, der in einer äußeren Ölhülle eingehüllt ist, wobei die Trägerflüssigkeit eine kontinuierliche wässrige Phase ist, wobei der innere wässrige Kern gegebenenfalls einen Durchmesser aufweist von: (a) 10 μm bis 500 μm; (b) 10 μm bis 200 μm; (c) 10 μm bis 150 μm; (d) 10 μm bis 100 μm; (e) 10 μm bis 50 μm; oder (f) etwa 100 μm und/oder wobei die äußere Ölhülle eine Dicke aufweist von: (a) 10 μm bis 200 μm; (b) 10 μm bis 200 μm; (c) 10 μm bis 150 μm; (d) 10 μm bis 100 μm; (e) 10 μm bis 50 μm; oder (f) etwa 100 μm.

5. Verfahren nach einem der Ansprüche 1-4, wobei die Trägerflüssigkeit eine mit Wasser nicht mischbare Flüssigkeit ist, gegebenenfalls wobei: (i) die mit Wasser nicht mischbare Flüssigkeit ein Öl ist, beispielsweise ausgewählt aus: (a) einem Kohlenwasserstofföl; (b) einem Fluorkohlenstofföl; (c) einem Esteröl; (d) einem Öl, das eine hohe Löslichkeit für biologische Komponenten der wässrigen Phase aufweist; (e) einem Öl, das eine molekulare Diffusion zwischen Mikrotröpfchen hemmt; (f) einem Öl, das hydrophob und lipophob ist; (g) einem Öl, das eine gute Löslichkeit für Gase aufweist; und/oder (h) Kombinationen von beliebigen zwei oder mehr der vorstehenden; und/oder (ii) die Mikrotröpfchen in einer W/O-Emulsion enthalten sind, wobei die Mikrotröpfchen eine wässrige, dispergierte Phase darstellen und die Trägerflüssigkeit eine kontinuierliche Ölphase darstellt.

6. Verfahren nach einem der Ansprüche 1-4, wobei die Trägerflüssigkeit eine wässrige Flüssigkeit, beispielsweise eine phosphatgepufferte Salzlösung (PBS) ist, und wobei die Mikrotröpfchen gegebenenfalls in einer W/O/W-Doppelemulsion enthalten sind, wobei die Mikrotröpfchen Folgendes umfassen: (a) einen inneren Kern aus einem wässrigen Wachstumsmedium, der in eine äußere Ölhülle eingehüllt ist, als die dispergierte Phase, und (b) die Trägerflüssigkeit als die kontinuierliche wässrige Phase.

7. Verfahren nach einem der vorhergehenden Ansprüche, wobei die Mikrotröpfchen in einer Emulsion enthalten sind, wobei die Trägerflüssigkeit die kontinuierliche Phase darstellt und die Mikrotröpfchen die dispergierte Phase darstellen, und wobei die Emulsion ferner ein Tensid und gegebenenfalls ein Co-Tensid umfasst, gegebenenfalls wobei:

(a) das Tensid und/oder das Co-Tensid an einer Schnittstelle der dispergierten und der kontinuierlichen Phase positioniert ist; und/oder (b) die Mikrotröpfchen in einer W/O/W-Doppelemulsion nach Anspruch 6 enthalten sind und das Tensid und/oder das Co-Tensid an der Schnittstelle zwischen dem wässrigen Kern und der Ölhülle positioniert ist; und/oder (c) das Tensid und/oder das Co-Tensid ein Fluortensid umfasst, das eine nichtionische Kopfgruppe aufweist; und/oder (d) das Tensid und/oder das Co-Tensid einen perfluorierten Polyether (PFPE) umfasst; und/oder (e) das Tensid und/oder das Co-Tensid ein PFPE-Polyethylenglycol-(PEG)-Blockcopolymer, zum Beispiel ein Diblock- oder ein Triblockcopolymer, zum Beispiel ein PFPE-PEG-PFPE-Triblockcopolymer umfasst.

8. Verfahren nach einem der vorhergehenden Ansprüche, wobei die Mikrotröpfchen monodispers sind.

9. Verfahren nach einem der vorhergehenden Ansprüche, wobei:

   (A) der Schritt (c) des gemeinsamen Einkapselns ein Mischen umfasst von: (a) dem Pool von mutierten Produzentenzellen; (b) einer Population der Zielzellen; (c) einem wässrigen Wachstumsmedium; (d) einer mit Wasser nicht mischbaren Flüssigkeit, zum Beispiel einem Öl nach Anspruch 5; und (e) einem Tensid, zum Beispiel nach Anspruch 7, unter Bedingungen, wobei eine einzelne Emulsion des Typen W/O gebildet wird, die Mikrotröpfchen des wässrigen Wachstumsmediums umfasst, die in der mit Wasser nicht mischbaren Flüssigkeit dispergiert sind, wobei nach dem Schritt (d) des Inkubierens gegebenenfalls, eine wässrige Trägerflüssigkeit, zum Beispiel nach Anspruch 6, mit der einzelnen Emulsion aus Schritt (c) unter Bedingungen gemischt wird, unter denen sich eine W/O/W-Doppelemulsion bildet, die Mikrotröpfchen des wässrigen Wachstumsmediums umfasst, die von der mit Wasser nicht mischbaren Flüssigkeit eingehüllt und in der wässrigen Trägerflüssigkeit dispergiert sind; oder
   (B) der Schritt (c) des gemeinsamen Einkapselns ein Mischen umfasst von: (a) dem Pool von mutierten Produzentenzellen; (b) einer Population der Zielzellen; (c) einem wässrigen Wachstumsmedium; (d) einer mit Wasser nicht mischbaren Flüssigkeit, zum Beispiel einem Öl nach Anspruch 5; (e) einem Tensid, zum Beispiel nach Anspruch 7; und (f) einer wässrigen Trägerflüssigkeit, zum Beispiel nach Anspruch 6 unter Bedingungen, unter denen sich eine W/O/W-Doppelemulsion bildet, die Mikrotröpfchen des wässrigen Wachstumsmediums umfasst, die von der mit Wasser nicht mischbaren Flüssigkeit eingehüllt und in der wässrigen Trägerflüssigkeit dispergiert sind;
   wobei das Mischen gegebenenfalls Folgendes umfasst: (a) Vortexen und/oder (b) Beschallung; (c) Homogenisierung; (d) Pico-Injektion und/oder (e) Strömungskonzentration, und/oder wobei der Schritt (c) des gemeinsamen Einkapselns ein Eliminieren von leeren Mikrotröpfchen umfasst, die keine mutierten Produzenten- und/oder Zielzelle(n) enthalten, zum Beispiel wobei die mutierten Produzentenzellen und/oder die Zielzellen fluoreszierend gekennzeichnet werden und leere Mikrotröpfchen durch eine FADS eliminiert werden.

10. Verfahren nach einem der vorhergehenden Ansprüche, wobei der Schritt (d) des Inkubierens ein Halten der Mikrotröpfchenbibliothek auf einer Temperatur von 15 °C-95 °C für mindestens 1 Stunde umfasst, wobei gegebenenfalls: (a) die Mikrotröpfchenbibliothek auf einer Temperatur gehalten wird von: (i) 15 °C-42 °C; (ii) 20 °C-40 °C; (iii) 20 °C-37 °C; (iv) 20 °C-30 °C; oder (v) etwa 25 °C; (vi) 40 °C-60 °C; (vii) 60 °C-80 °C; oder (viii) 80 °C-98 °C; (b) der Schritt (d) des Inkubierens ein Halten der Mikrotröpfchenbibliothek auf der Temperatur für etwa 2, 4, 6, 12, 24 oder 48 Stunden oder für bis zu 7 Tage, zum Beispiel für 1, 2, 3, 4, 5, 6 oder 7 Tage umfasst; (c) der Schritt (d) des Inkubierens ein Halten des Mikrotröpfchenbibliothek auf der Temperatur für bis zu 2 Wochen, zum Beispiel für 1 Woche oder 2 Wochen umfasst.

11. Verfahren nach einem der vorhergehenden Ansprüche, wobei:

   (A) der Schritt (e) des Screenings Folgendes umfasst: (i) Eliminieren von Mikrotröpfchen, die Zielzellen enthalten, wobei die Zielzellen gegebenenfalls fluoreszierend gekennzeichnet sind, und wobei Mikrotröpfchen, die Zielzellen enthalten, durch eine FADS eliminiert werden; und/oder (ii) eine FADS-Sortierung nach Mikrotröpfchen umfasst, in denen die mutierten Produzentenzellen über die Zielzellen hinausgewachsen sind oder diese bis zur Extinktion überwuchert haben; und/oder
   (B) das Verfahren ferner einen Potenzanalyse-Schritt umfasst, der während des Schritts (d) des Inkubierens ausgeführt wird, wobei die relative Wachstumsrate in der gemeinsamen Kultivierung von mutierten Produzentenzellen und Zielzellen bestimmt wird, wobei gegebenenfalls: (i) der Potenzanalyseschritt ein Auswählen durch eine FADS von Mikrotröpfchen umfasst, die ausschließlich mutierte Produzentenzellen enthalten, zum Beispiel wobei zwei oder mehr aufeinanderfolgende Runden einer FADS-Auswahl während des Inkubierens ausgeführt werden;
   und/oder (ii) Zielzellen fluoreszierend gekennzeichnet werden und Mikrotröpfchen, die Zielzellen enthalten, einer fortgesetzten Inkubation unterzogen werden.

12. Verfahren nach einem der vorhergehenden Ansprüche, wobei:

(A) die prokaryotische Produzentenspezies ausgewählt ist aus:

Stämmen von Archaea, zum Beispiel ausgewählt aus den Phyla-Stämmen: (a) Crenarchaeota; (b) Euryarchaeota; (c) Korarchaeota; (d) Nanoarchaeota und (e) Thaumarchaeota, zum Beispiel *Haloferax volcanii* oder *Sulfolobus* spp.;
und/oder Bakterien, zum Beispiel ausgewählt aus: (a) Aktinomyceten; (b) *Pseudomonas* spp., und (c) *Bacillus* spp., gegebenenfalls ausgewählt aus *Streptomyces* spp., zum Beispiel *Streptomyces coelicolor; Streptomyces lividans; Streptomyces venezuelae; Streptomyces griseus; Streptomyces avermetilis; Streptomyces bingchenggensis; Kutzneria albida; Saccaropolyspora erythrea; Mycobacterium marinum; Bacillus amyloliquefaciens* subsp. *Plantarum;* und *Micromonospora echinospora,* und/oder

(B) die Zielzelle eine Bakterien- oder eukaryotische Zelle ist, und gegebenenfalls eine Pilzzelle; eine Säugetierzelle; eine höhere Pflanzenzelle, eine Protozoonzelle, eine Hellminthenzelle, eine Algenzelle; oder eine Wirbellosen-Zelle, zum Beispiel eine Krebszelle, zum Beispiel eine humane Krebszelle oder ein pathogenes Bakterium ist, zum Beispiel ausgewählt aus: *Pseudomonas aeruginosa, Acinetobacter baumannii, Escherichia coli, Klebsiella pneumaniae, Staphylococcus aureus, Enterococcus faecium* und *Enterobacter* spp.

13. Verfahren nach einem der vorhergehenden Ansprüche, wobei:

(A) die mutierte Produzentenzelle und/oder die Zielzelle(n) aus Schritt (c) gekennzeichnet sind, z. B. durch eine fluoreszierende Kennzeichnung, und/oder wobei der Schritt (e) des Screenings eine FADS umfasst; und/oder
(B) der Pool von mutierten Produzentenzellen Folgendes umfasst: (a) mindestens $0,5 \times 10^5$ mutierte Zellen, zum Beispiel mindestens $1 \times 10^5$ mutierte Zellen; (b) mindestens $5 \times 10^5$ mutierte Zellen; (c) mindestens $1 \times 10^6$ mutierte Zellen; (d) $0,5 \times 10^6$ bis $2 \times 10^6$ mutierte Zellen; (e) etwa $1 \times 10^6$ mutierte Zellen; oder (f) bis zu $10 \times 10^6$ mutierte Zellen; und/oder
(C) der Schritt (b) der Transposon-Mutagenese eine Einfügungsrate erreicht von: (a) mindestens einem Transposon pro 50 Basenpaare von Bakterien-DNA; (b) mindestens ein Transposon pro 30 Basenpaare von Bakterien-DNA; (c) mindestens ein Transposon pro 25 Basenpaare von Bakterien-DNA; (d) mindestens ein Transposon pro 15 Basenpaare von Bakterien-DNA; (e) mindestens ein Transposon pro 10 Basenpaare von Bakterien-DNA; oder (f) mindestens ein Transposon pro 5 Basenpaare von Bakterien-DNA; und/oder
(D) der Schritt (b) der Transposon-Mutagenese eine Einfügung des $Tn_A$ umfasst in: (a) chromosomale (genomische) DNA; (b) Plasmid-DNA, oder (c) eine Mischung aus chromosomaler (genomischer) und Plasmid-DNA der Zellen der Produzenten-Bakterienspezies; und/oder
(E) der Schritt (b) der Transposon-Mutagenese *in vivo* oder *in vitro* auftritt; und/oder
(F) das zytotoxische Mittel ausgewählt ist aus: (a) einem Antibiotikum; (b) einem antineoplastischen Mittel; und (c) einem antimykotischen Mittel; und/oder
(G) in dem Schritt (b) die $Tn_A$-Einfügung in zelluläre DNA der prokaryotischen Produzentenspezies :

(a) stromaufwärts eines Gens oder eines Operons eines kryptischen zytotoxischen Mittels auftritt, wobei der $Tn_AP$ eine erhöhte Transkription antreibt und so zur Expression des Gens oder des Operons des kryptischen zytotoxischen Mittels führt;
(b) stromaufwärts eines Aktivators eines Gens oder eines Operons eines kryptischen zytotoxischen Mittels auftritt, wobei der $Tn_AP$ eine erhöhte Transkription des Aktivators antreibt, der *in trans* wirkt, um die Expression des Gens oder des Operons des kryptischen zytotoxischen Mittels zu erhöhen;
(c) durch eine Einfügung einen Repressor eines Gens oder eines Operons eines kryptischen zytotoxischen Mittels deaktiviert, wodurch sich die Expression des Gens oder des Operons des kryptischen zytotoxischen Mittels erhöht;
(d) stromaufwärts eines Enzyms auftritt, das in eine Synthese eines zytotoxischen Mittels involviert ist, wobei der $Tn_AP$ eine erhöhte Transkription antreibt und so zu einer erhöhten Expression des Enzyms führt;
(e) durch eine Einfügung ein Enzym deaktiviert, für das ein zytotoxisches Mittel ein Substrat ist, wodurch sich das Niveau des zytotoxischen Mittels erhöht;
(f) stromaufwärts eines Enzyms auftritt, das als eine untergeordnete Tätigkeit ein zytotoxisches Mittel produziert, wobei der $Tn_AP$ eine erhöhte Transkription antreibt und so zu einer erhöhten Expression des Enzyms führt, wodurch er dessen untergeordnete Tätigkeit erhöht und das Niveau des zytotoxischen Mittels erhöht;

(g) durch eine Einfügung eine oder mehrere Domänen eines Multidomänenenzyms deaktiviert, wodurch sie dessen Funktion derartig verändert, dass es direkt oder indirekt ein zytotoxisches Mittel synthetisiert;

(h) stromaufwärts von einer oder mehreren Domänen eines Multidomänenenzyms auftritt, wobei der Tn$_A$P eine erhöhte Transkription der einen oder mehreren Domänen antreibt, wodurch er die Funktion des Enzyms derartig verändert, dass es direkt oder indirekt ein zytotoxisches Mittel synthetisiert; oder

(i) ein Codierungsrepertoire derartig verändert, das ein zytotoxisches Mittel direkt oder indirekt exprimiert wird.

14. Verfahren zum Identifizieren eines zytotoxischen Mittels, das ein Screening nach mutierten Bakterien umfasst, um Produzenten eines zytotoxischen Mittels zu identifizieren, das gegen eine Zielzelle aktiv ist, nach einem Verfahren nach einem der vorhergehenden Ansprüche.

15. Prozess zur Herstellung eines zytotoxischen Mittels, der das Verfahren nach Anspruch 14 umfasst, ferner umfassend ein Synthetisieren oder ein Isolieren des zytotoxischen Mittels von den mutierten Bakterien, und danach gegebenenfalls ein Mischen des synthetisierten oder isolierten zytotoxischen Mittels mit einem pharmazeutisch unbedenklichen Hilfsstoff, um eine pharmazeutische Zusammensetzung zu produzieren.

**Revendications**

1. Procédé de criblage de cellules procaryotes mutantes pour identifier des producteurs d'un agent cytotoxiques actif contre une cellule cible, le procédé comprenant les étapes de :

(a) fourniture de cellules d'une espèce procaryote productrice ;

(b) génération d'un groupe de cellules productrices mutantes par mutagenèse par transposon des cellules de l'étape (a) avec un transposon d'activation (Tn$_A$), ledit Tn$_A$ comprenant un promoteur tourné vers l'extérieur (Tn$_A$P) capable d'augmenter la transcription d'un gène au niveau ou à proximité de son site d'insertion dans l'ADN desdites cellules productrices ;

(c) co-encapsulation d'éléments individuels du groupe de l'étape (b) avec une ou plusieurs cellules cibles dans des microgouttelettes, les microgouttelettes comprenant un volume de milieu de croissance aqueux en suspension dans un liquide porteur non miscible, générant ainsi une banque de microgouttelettes comprenant chacune une cellule productrice mutante unique et une ou plusieurs cellules cibles ;

(d) incubation de la banque de microgouttelettes de l'étape (c) dans des conditions appropriées pour la co-culture de la cellule productrice mutante unique et de la ou des cellules cibles pour produire une banque de microcultures, les cellules productrices mutantes produisant ainsi un agent cytotoxique actif contre la ou les cellules cibles devenant trop grandes pour les cellules cibles dans chaque microculture ; et

(e) criblage de la banque de microcultures de l'étape (d) pour identifier les microcultures dans lesquelles les cellules cibles ont été dépassées en taille ou envahies jusqu'à extinction par des cellules productrices mutantes.

2. Procédé selon la revendication 1 comprenant en outre l'étape de séquençage de l'ADN de cellules productrices mutantes dans des microgouttelettes dans lesquelles des cellules cibles ont été dépassées en taille ou envahies jusqu'à extinction par des cellules productrices mutantes, éventuellement : (i) l'ADN adjacent ou proche du site d'insertion du TnA étant séquencé, par exemple par un séquençage qui comprend l'amplification sélective de jonctions transposon-ADN cellulaire ; et/ou (ii) le séquençage comprenant un séquençage massivement parallèle à haut débit, par exemple choisi parmi : (a) la biochimie de séquençage par synthèse (SBS) ; et/ou (b) le séquençage par nanopores ; et/ou (c) le séquençage par courant à effet tunnel ; et/ou le (d) pyroséquençage ; et/ou (e) le séquençage par ligature (séquençage SOLiD) ; et/ou (f) un semiconducteur ionique ; et/ou (g) le séquençage par spectrométrie de masse ; et/ou (iii) environ 25, 50, 75, 100 ou plus de 100 paires de bases d'ADN adjacent ou proche du site d'insertion de TnA étant séquencées ; et/ou (iv) l'ADN séquencé étant en 5' et/ou en 3' par rapport au site d'insertion de TnA.

3. Procédé selon la revendication 1 ou 2 comprenant en outre l'étape de séquençage de transcrits d'ARNm produits par le TnAP dans des cellules productrices mutantes dans des microgouttelettes dans lesquelles des cellules cibles ont été dépassées en taille ou envahies jusqu'à extinction par des cellules productrices mutantes pour produire un profil de transcrit d'ARNm, éventuellement ledit profil de transcrit d'ARNm comprenant la détermination :

(a) des séquences desdits transcrits d'ARNm produits par le Tn$_A$P ; et/ou

(b) du début et de la fin des transcrits d'ARNm produits par le TnAP ; et/ou

(c) des longueurs desdits transcrits d'ARNm produits par le Tn$_A$P ; et/ou

(d) de l'abondance relative desdits transcrits d'ARNm produits par le Tn$_A$P ; et/ou

(e) du site de transcription sur l'ADN cellulaire ; et/ou

(f) pour savoir si les transcrits d'ARNm produits par le Tn$_A$P sont sens ou antisens par rapport à l'ADN cellulaire ; et/ou

(g) pour savoir si les transcrits d'ARNm produits par le Tn$_A$P correspondent aux ORF par rapport à l'ADN cellulaire ; et/ou

(h) pour savoir si les transcrits d'ARNm produits par le Tn$_A$P codent des domaines de protéines et/ou des protéines procaryotes.

4. Procédé selon l'une quelconque des revendications précédentes, lesdites microgouttelettes : (a) étant sensiblement sphériques et présentant un diamètre de : (a) 10 $\mu$m à 500 $\mu$m ; (b) 10 $\mu$m à 200 $\mu$m ; (c) 10 $\mu$m à 150 $\mu$m ; (d) 10 $\mu$m à 100 $\mu$m ; (e) 10 $\mu$m à 50 $\mu$m ; ou (f) environ 100 $\mu$m ; et/ou (b) comprenant un volume de milieu de croissance aqueux à l'état de liquide ou de gel ; et/ou (c) comprenant un noyau interne de milieu de croissance aqueux enveloppé dans une enveloppe d'huile externe, le liquide porteur étant une phase aqueuse continue, éventuellement ledit noyau aqueux interne présentant un diamètre de : (a) 10 $\mu$m à 500 $\mu$m ; (b) 10 $\mu$m à 200 $\mu$m ; (c) 10 $\mu$m à 150 $\mu$m ; (d) 10 $\mu$m à 100 $\mu$m ; (e) 10 $\mu$m à 50 $\mu$m ; ou (f) environ 100 $\mu$m et/ou ladite enveloppe d'huile externe présentant une épaisseur de : (a) 10 $\mu$m à 200 $\mu$m ; (b) 10 $\mu$m à 200 $\mu$m ; (c) 10 $\mu$m à 150 $\mu$m ; (d) 10 $\mu$m à 100 $\mu$m ; (e) 10 $\mu$m à 50 $\mu$m ; ou (f) environ 100 $\mu$m.

5. Procédé selon l'une quelconque des revendications 1 à 4, ledit liquide porteur étant un liquide non miscible dans l'eau, éventuellement : (i) ledit liquide non miscible dans l'eau étant une huile, par exemple choisie parmi : (a) une huile hydrocarbonée ; (b) une huile fluorocarbonée ; (c) une huile d'ester ; (d) une huile présentant une faible solubilité pour les composants biologiques de la phase aqueuse ; (e) une huile qui empêche la diffusion moléculaire entre les microgouttelettes ; (f) une huile qui est hydrophobe et lipophobe ; (g) une huile présentant une bonne solubilité pour les gaz ; et/ou (h) des combinaisons de deux quelconques des éléments précédents ou plus ; et/ou (ii) lesdites microgouttelettes étant comprises dans une émulsion eau/huile dans laquelle les microgouttelettes constituent une phase aqueuse dispersée et le liquide porteur constitue une phase huileuse continue.

6. Procédé selon l'une quelconque des revendications 1 à 4, ledit liquide porteur étant un liquide aqueux, par exemple une solution saline tamponnée au phosphate (PBS), et les microgouttelettes étant éventuellement comprises dans une émulsion double eau/huile/eau dans laquelle les microgouttelettes comprennent : (a) un noyau interne de milieu de croissance aqueux enveloppé dans une enveloppe d'huile externe en tant que phase dispersée, et (b) le liquide porteur en tant que phase aqueuse continue.

7. Procédé selon l'une quelconque des revendications précédentes, lesdites microgouttelettes étant comprises dans une émulsion, ledit liquide porteur constituant la phase continue et lesdites microgouttelettes constituant la phase dispersée, et ladite émulsion comprenant en outre un tensioactif et éventuellement un co-tensioactif, éventuellement : (a) ledit tensioactif et/ou co-tensioactif se trouvant au niveau de l'interface des phases continue et dispersée ; et/ou (b) lesdites microgouttelettes étant comprises dans une émulsion double eau/huile/eau telle que définie dans la revendication 6 et ledit tensioactif et/ou co-tensioactif se trouvant au niveau de l'interface du noyau aqueux et de l'enveloppe d'huile ; et/ou (c) ledit tensioactif et/ou co-tensioactif comprenant un tensioactif fluoré comportant un groupe de tête non ionique ; et/ou (d) ledit tensioactif et/ou co-tensioactif comprenant un polyéther perfluoré (PFPE) ; et/ou (e) ledit tensioactif et/ou co-tensioactif comprenant un copolymère séquencé PFPE-polyéthylène glycol (PEG), par exemple un copolymère biséquencé ou triséquencé, par exemple un copolymère triséquencé PFPE-PEG-PFPE.

8. Procédé selon l'une quelconque des revendications précédentes, lesdites microgouttelettes étant monodispersées.

9. Procédé selon l'une quelconque des revendications précédentes :

(A) ladite étape de co-encapsulation (c) comprenant le mélange : (a) du groupe de cellules productrices mutantes ; (b) d'une population de cellules cibles ; (c) d'un milieu de croissance aqueux ; (d) d'un liquide non miscible dans l'eau, par exemple une huile telle que définie dans la revendication 5 ; et (e) d'un tensioactif, par exemple tel que défini dans la revendication 7, dans des conditions grâce auxquelles une émulsion unique de type eau/huile comprenant des microgouttelettes du milieu de croissance aqueux dispersées dans le liquide non miscible dans l'eau est formée, éventuellement suite à l'étape (d) d'incubation, un liquide porteur aqueux, par exemple tel que défini dans la revendication 6, étant mélangé à l'émulsion unique de l'étape (c) dans des conditions grâce auxquelles une émulsion double eau/huile/eau comprenant des microgouttelettes du milieu de croissance aqueux enveloppées dans le liquide non miscible dans l'eau et dispersées dans le liquide porteur aqueux est formée ; ou

(B) ladite étape (c) de co-encapsulation comprenant le mélange : (a) du groupe de cellules productrices mutantes ; (b) d'une population de cellules cibles ; (c) d'un milieu de croissance aqueux ; (d) d'un liquide non miscible dans l'eau, par exemple une huile telle que définie dans la revendication 5 ; et (e) d'un tensioactif, par exemple tel que défini dans la revendication 7, et (f) d'un liquide porteur aqueux, par exemple tel que défini dans la revendication 6, dans des conditions grâce auxquelles une émulsion double eau/huile/eau comprenant des microgouttelettes du milieu de croissance aqueux enveloppées dans le liquide non miscible dans l'eau et dispersées dans le liquide porteur aqueux est formée ;

éventuellement ledit mélange comprenant : (a) une agitation au vortex et/ou (b) une sonication ; (c) une homogénéisation ; (d) une pico-injection et/ou (e) une focalisation de flux et/ou ladite étape (c) de co-encapsulation comprenant en outre l'élimination des microgouttelettes vides qui ne contiennent pas de cellule(s) productrice(s) mutante(s) et/ou cible(s), par exemple lesdites cellules productrices mutantes et/ou cellules cibles étant marquées par fluorescence et lesdites microgouttelettes vides étant éliminées par tri de gouttes activé par fluorescence (FADS).

10. Procédé selon l'une quelconque des revendications précédentes, ladite étape (d) d'incubation comprenant le maintien de la banque de microgouttelettes à une température de 15°C à 95°C pendant au moins 1 heure, éventuellement : (a) ladite banque de microgouttelettes étant maintenue à une température de : (i) 15°C à 42°C ; (ii) 20°C à 40°C ; (iii) 20°C à 37°C ; (iv) 20°C à 30°C ; ou (v) environ 25°C ; (vi) 40°C à 60°C ; (vii) 60°C à 80°C ; ou (viii) 80°C à 98°C ; (b) ladite étape (d) d'incubation comprenant le maintien de la banque de microgouttelettes à ladite température pendant environ 2, 4, 6, 12, 24 ou 48 heures ou pendant jusqu'à 7 jours, par exemple pendant 1, 2, 3, 4, 5, 6 ou 7 jours; (c) ladite étape (d) d'incubation comprenant le maintien de la banque de microgouttelettes à ladite température pendant jusqu'à 2 semaines, par exemple pendant 1 semaine ou 2 semaines.

11. Procédé selon l'une quelconque des revendications précédentes :

(A) ladite étape (e) de criblage comprenant : (i) l'élimination des gouttelettes qui contiennent des cellules cibles, éventuellement lesdites cellules cibles étant marquées par fluorescence et les microgouttelettes qui contiennent des cellules cibles étant éliminées par FADS ; et/ou (ii) comprenant un tri FADS pour les micro gouttelettes dans lesquelles des cellules cibles ont été dépassées en taille ou envahies jusqu'à extinction par des cellules productrices mutantes ; et/ou

(B) ledit procédé comprenant en outre une étape d'analyse de puissance effectuée pendant l'étape (d) d'incubation ce qui permet de déterminer le taux de croissance relatif dans une co-culture de cellules productrices mutantes et de cellules cibles, éventuellement : (i) ladite étape d'analyse de puissance comprenant la sélection de micro gouttelettes qui contiennent uniquement des cellules productrices mutantes par FADS, par exemple deux cycles successifs de sélection par FADS ou plus étant effectués pendant l'incubation ; et/ou (ii) des cellules cibles étant marquées par fluorescence et les microgouttelettes qui contiennent des cellules cibles étant soumises à une incubation continue.

12. Procédé selon l'une quelconque des revendications précédentes :

(A) l'espèce procaryote productrice étant choisie parmi :

les archées, par exemple choisie parmi les phylums : (a) les Crenarchaeota ; (b) les Euryarchaeota ; (c) les Korarchaeota ; (d) les Nanoarchaeota et (e) les Thaumarchaeota, par exemple *Haloferax volcanii* ou *Sulfolobus* spp. ; et/ou

les bactéries, par exemple choisie parmi : (a) les actinomycètes ; (b) *Pseudomonas* spp., et (c) *Bacillus* spp., éventuellement choisie parmi *Streptomyces* spp, par exemple *Streptomyces coelicolor* ; *Streptomyces lividans* ; *Streptomyces venezuealae* ; *Streptomyces griseus* ; *Streptomyces avermetilis* ; *Streptomyces bingchenggensis* ; *Kutzneria albida* ; *Saccaropolyspora erythrea* ; *Mycobacterium marinum* ; *Bacillus amyloliquefaciens* subsp. *Plantarum* et *Micromonospora echinospora* ;

et/ou

(B) ladite cellule cible étant une cellule bactérienne ou eucaryote, éventuellement étant fongique ; de mammifère ; une cellule végétale supérieure ; protozoaire ; une cellule d'helminthe ; algale ; ou une cellule d'invertébré, par exemple une cellule cancéreuse, par exemple une cellule cancéreuse humaine, ou une bactérie pathogène, par exemple choisie parmi : *Pseudomonas aeruginosa, Acinetobacter baumannii, Escherichia coli, Klebsiella pneumoniae, Staphylococcus aureus, Enterococcus faecium, Enterococcus faecalis* et *Enterobacter* spp.

13. Procédé selon l'une quelconque des revendications précédentes :

   (A) ladite cellule productrice mutante et/ou ladite ou lesdites cellules cibles de l'étape (c) étant marquées, par exemple avec un marqueur fluorescent et/ou ladite étant (e) de criblage comprenant un FADS ; et/ou
   (B) ledit groupe de cellules productrices mutantes comprenant : (a) au moins 0,5 x 10$^5$ mutants, par exemple au moins 1 x 10$^5$ mutants ; (b) au moins 5 x 10$^5$ mutants ; (c) au moins 1 x 10$^6$ mutants ; (d) 0,5 x 10$^6$ à 2 x 10$^6$ mutants ; (e) environ 1 x 10$^6$ mutants ; ou (f) jusqu'à 10 x 10$^6$ mutants ; et/ou
   (C) ladite étape (b) de mutagenèse par transposon produisant un taux d'insertion de : (a) au moins un transposon pour 50 paires de bases d'ADN bactérien ; (b) au moins un transposon pour 30 paires de bases d'ADN bactérien ; (c) au moins un transposon pour 25 paires de bases d'ADN bactérien ; (d) au moins un transposon pour 15 paires de bases d'ADN bactérien ; (e) au moins un transposon pour 10 paires de bases d'ADN bactérien ; ou (f) au moins un transposon pour 5 paires de bases d'ADN bactérien ; et/ou
   (D) ladite étape (b) de mutagenèse par transposon comprenant l'insertion du Tn$_A$ dans : (a) l'ADN chromosomique (génomique) ; (b) l'ADN plasmidique, ou (c) un mélange de l'ADN chromosomique (génomique) et plasmidique des cellules de l'espèce bactérienne productrice ; et/ou
   (E) ladite mutagenèse par transposon de l'étape (b) ayant lieu *in vivo* ou *in vitro* ; et/ou
   (F) ledit agent cytotoxique étant choisi parmi : (a) un antibiotique ; (b) un agent antinéoplasique ; et (c) un agent antimycotique ; et/ou
   (G) dans l'étape (b), ladite insertion de Tn$_A$ dans l'ADN cellulaire des espèces procaryotes productrices :

      (a) ayant lieu en amont d'un gène ou opéron d'agent cytotoxique cryptique, au niveau duquel le Tn$_A$P dirige la transcription accrue et conduit ainsi à l'expression du gène ou opéron d'agent cytotoxique cryptique ;
      (b) ayant lieu en amont d'un activateur d'un gène ou opéron d'agent cytotoxique cryptique, au niveau duquel le Tn$_A$P dirige la transcription accrue de l'activateur qui agit en trans pour augmenter l'expression du gène ou opéron d'agent cytotoxique cryptique ;
      (c) inactivant par insertion un répresseur d'un gène ou opéron d'agent cytotoxique cryptique, augmentant ainsi l'expression du gène ou opéron d'agent cytotoxique cryptique ;
      (d) ayant lieu en amont d'une enzyme impliquée dans une synthèse d'agent cytotoxique, au niveau duquel le Tn$_A$P dirige la transcription accrue et conduit ainsi à l'expression accrue de ladite enzyme ;
      (e) inactivant par insertion une enzyme pour laquelle un agent cytotoxique est un substrat, augmentant ainsi les taux de l'agent cytotoxique ;
      (f) ayant lieu en amont d'une enzyme qui produit un agent cytotoxique an tant qu'activité secondaire, au niveau de laquelle le Tn$_A$P dirige la transcription accrue et conduit ainsi à l'expression accrue de ladite enzyme, augmentant ainsi son activité secondaire et les taux de l'agent cytotoxique ;
      (g) inactivant par insertion un ou plusieurs domaines d'une enzyme multi-domaine, modifiant ainsi sa fonction de sorte qu'elle synthétise, directement ou indirectement, un agent cytotoxique ;
      (h) ayant lieu en amont d'un ou plusieurs domaine d'une enzyme multi-domaine, au niveau desquels le Tn$_A$P dirige la transcription accrue desdits un ou plusieurs domaines, modifiant ainsi la fonction de ladite enzyme de sorte qu'elle synthétise, directement ou indirectement, un agent cytotoxique ; ou
      (i) modifiant un répertoire de codage de sorte qu'un agent cytotoxique soit directement ou indirectement exprimé.

14. Procédé d'identification d'un agent cytotoxique comprenant le criblage de bactéries mutantes pour identifier des producteurs d'un agent cytotoxique actif contre une cellule cible selon un procédé tel que défini dans l'une quelconque des revendications précédentes.

15. Procédé de production d'un agent cytotoxique comprenant le procédé tel que défini dans la revendication 14, comprenant en outre la synthèse ou l'isolement dudit agent cytotoxique desdites bactéries mutantes, et ensuite éventuellement le mélange de l'agent cytotoxique isolé ou synthétisé avec un excipient pharmaceutiquement acceptable pour produire une composition pharmaceutique.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2014072697 A
- GB 2505819 A
- WO 0107651 A
- WO 9841869 A
- WO 9623810 A


**Non-patent literature cited in the description**

- **AHARONI et al.** High-Throughput Screening of Enzyme Libraries: Thiolactonases Evolved by Fluorescence-Activated Sorting of Single Cells in Emulsion Compartments. *Chemistry and Biology,* vol. 12 (12), 1281-1289
- **BERNATH et al.** In vitro compartmentalization by double emulsions: sorting and gene enrichment by fluorescence activated cell sorting. *Analytical Biochemistry,* vol. 325 (1), 151-157
- **BARET et al.** Fluorescence-activated droplet sorting (FADS): efficient microfluidic cell sorting based on enzymatic activity. *Lab on a Chip,* vol. 9 (13), 1850-1858
- **TROESCHEL et al.** *Methods Mol Biol.,* 2010, vol. 668, 117-39
- **KIM et al.** *Curr Microbiol.,* 2008, vol. 57 (4), 391-394
- **RHODIUS et al.** *Nucleic Acids Research,* 2011, 1-18
- **ZHAO et al.** *ACS Synth. Biol.,* 2013, vol. 2 (11), 662-669
- **SHAO et al.** *ACS Synth. Biol.,* 2013, vol. 2 (11), 662-669
- **WAGNER et al.** *J. Biotechnol.,* 2009, vol. 142, 200-204
- **SEGHEZZI et al.** *Applied Microbiology and Biotechnology,* 2011, vol. 90 (2), 615-623
- **WANG et al.** *Applied and Environmental Microbiology,* 2013, vol. 79 (14), 4484-4492
- **KIM et al.** *Biotechnology and Bioprocess Engineering,* 2008, vol. 13 (3), 313-318
- **VAN OPIJNEN et al.** *Nat. Methods,* 2009, vol. 6, 767-772
- **GAWRONSKI et al.** *PNAS,* 2009, vol. 106, 16422-16427
- **GOODMAN et al.** *Cell Host Microbe,* 2009, vol. 6, 279-289
- **LANGRIDGE et al.** *Genome Research,* 2009, vol. 19, 2308-2316
- **GALLAGHER et al.** *mBio,* 2011, vol. 2 (1), e00315-10
- **ALTSCHUL et al.** *J. Mol. Biol.,* 1990, vol. 215, 403-410
- **ALTSCHUL et al.** *Nucl. Acids Res.,* 1997, vol. 25, 3389-3402
- **BERNATH et al.** *Analytical Biochemistry,* 2004, vol. 325, 151-157
- **HOLTZE ; WEITZ.** *Lab Chip,* 2008, vol. 8 (10), 1632-1639
- **HOLTZE et al.** *Lab Chip.,* 2008, vol. 8 (10), 1632-1639
- **ANNA et al.** *Appl. Phys. Lett.,* 2003, vol. 82 (3), 364-366
- **BARET et al.** *Lab Chip,* 2009, vol. 9, 1850-1858
- **CHALFIE et al.** *Science,* 1994, vol. 263, 802-805
- **HEIM et al.** *Nature,* 1995, vol. 373, 663-4